# EUROPEAN PATENT SPECIFICATION

(11) **EP 4 318 392 B1**
(45) Date of publication and mention of the grant of the patent: **19.08.2026**
(21) Application number: 22780964.7
(22) Date of filing: 29.03.2022
(51) Int. Cl.: G06T 7/00, A61B 6/00, A61B 6/58

(54) **FAILED PHOTOGRAPH IMAGE MANAGEMENT DEVICE, METHOD FOR OPERATING FAILED PHOTOGRAPH IMAGE MANAGEMENT DEVICE, PROGRAM FOR OPERATING FAILED PHOTOGRAPH IMAGE MANAGEMENT DEVICE, AND RADIOGRAPHIC IMAGE CAPTURING SYSTEM**
FEHLGESCHLAGENE FOTOGRAFIENBILDVERWALTUNGSVORRICHTUNG, VERFAHREN ZUM BETRIEB DER FEHLGESCHLAGENEN FOTOGRAFIENBILDVERWALTUNGSVORRICHTUNG UND RÖNTGENBILDERFASSUNGSSYSTEM
DISPOSITIF DE GESTION D'IMAGE PHOTOGRAPHIQUE RATÉE, PROCÉDÉ DE FONCTIONNEMENT DE DISPOSITIF DE GESTION D'IMAGE PHOTOGRAPHIQUE RATÉE, PROGRAMME DE FONCTIONNEMENT DE DISPOSITIF DE GESTION D'IMAGE PHOTOGRAPHIQUE RATÉE, ET SYSTÈME DE CAPTURE D'IMAGE RADIOGRAPHIQUE

(30) Priority: 30.03.2021 JP 2021057320
(43) Date of publication of application: 07.02.2024
(73) Proprietor: FUJIFILM Corporation, Tokyo 106-8620 (JP)
(72) Inventor: SUGAHARA, Masataka, Ashigarakami-gun, Kanagawa 258-8538 (JP)
(74) Representative: Dehns Germany Partnerschaft mbB
(86) International application number: PCT/JP2022/015617
(87) International publication number: WO 2022/210753

(56) References cited:
- WO-A1-2020/209290
- JP-A- 2006 218 139
- JP-A- 2020 025 781
- JP-A- 2020 025 781
- JP-A- 2020 199 163
- JP-A- 2022 035 719
- US-A1- 2019 236 773
- US-A1- 2019 236 773

## Description

### BACKGROUND OF THE INVENTION

### 1. Field of the Invention

The technology of the present disclosure relates to an imaging failure image management apparatus, an operation method and an operation program of an imaging failure image management apparatus, and a radiography system.

### 2. Description of the Related Art

A radiography apparatus that captures a radiation image of a subject is known in the medical field. In the field of radiography, a failure in imaging is generally referred to as "imaging failure", and a radiation image in which imaging has failed is referred to as "imaging failure image". Reasons for determining the imaging failure include inappropriate positioning of an imaging part and insufficient dose of radiation. In the radiography apparatus, the captured radiation image is displayed on a console immediately after the radiography. A technician confirms the radiation image displayed on the console, determines whether or not the imaging failure has occurred, and performs re-imaging in a case in which the imaging failure has occurred.

JP2020-025781A describes a radiography apparatus on which an imaging failure determination device (corresponding to a determination unit in JP2020-025781A) configured by a machine learning model is mounted. The imaging failure determination device receives a radiation image captured by the radiography apparatus as input, and outputs a determination result as to whether or not the input radiation image is an imaging failure image. The radiography apparatus described in JP2020-025781A is provided with a function of correcting the determination result output by the imaging failure determination device by an operator, such as the technician, of the radiography apparatus, and additionally training the imaging failure determination device by using training data using the corrected determination result.

### SUMMARY OF THE INVENTION

Since the imaging failure determination device is configured by the machine learning model, in general, the determination accuracy is further improved as the number of the training data is larger. Therefore, in some cases, it is possible to improve the determination accuracy of the imaging failure determination device by additionally training the imaging failure determination device, which is trained once, by using new training data.

However, in the radiography apparatus described in JP2020-025781A, the imaging failure determination device is additionally trained by using the determination result corrected by the determination of the operator of the radiography apparatus as the training data. Therefore, in a case in which the determination of the operator is incorrect, the imaging failure determination device is additionally trained by using the training data in which the determination result is incorrect, and there is a concern that the determination accuracy of the imaging failure determination device is decreased.

The technology of the present disclosure provides an imaging failure image management apparatus, an operation method and an operation program of an imaging failure image management apparatus, and a radiography system capable of suppressing a decrease in determination accuracy of an imaging failure determination device mounted on a radiography apparatus.

In order to achieve the above object, the technology of the present disclosure relates to an imaging failure image management apparatus as defined by claim 1.

In a case in which both a primary determination result, which is the determination result output by the first imaging failure determination device, and a secondary determination result, which is a determination result by an operator of the radiography apparatus, are included in the data set, the processor may display the primary determination result and the secondary determination result in a distinguishable manner in the display control processing.

The processor may acquire a data set including an imaging failure determination result that there is a possibility that the radiation image corresponds to the imaging failure image in at least one of the primary determination result or the secondary determination result in the data set in the data set acquisition processing.

In a case in which an approval instruction by a user is input for the determination result of the displayed data set, the processor may record approval information indicating that approval is made, in association with the data set which is an approval target.

In a case in which a review by a user is performed for the determination result, the processor may record information indicating that the review is performed, in association with the data set which is a review target.

The processor may be able to accumulate the training data in a storage unit.

The processor may not execute the training processing while an accumulated number of the training data is less than a set number set in advance, and may execute the training processing in a case in which the accumulated number reaches the set number.

In a case in which the training processing is executed for the second imaging failure determination device, the processor may transmit an update notification indicating that the first imaging failure determination device is updatable by the second imaging failure determination device to the radiography apparatus.

The update notification may be a trigger for the radiography apparatus to start update processing of the first imaging failure determination device, or a notification that is displayable on a console of the radiography apparatus.

The processor may store the second imaging failure determination device for which the training processing is executed in a storage unit accessible to the radiography apparatus.

The processor may execute test processing using test data for the second imaging failure determination device for which the training processing is executed, and may be able to perform control of displaying a test result on the display.

The second imaging failure determination device immediately before the training processing is executed may be the same as the first imaging failure determination device, and the processor may execute additional training for the second imaging failure determination device.

Also provided is a method as defined by claim 13.

Also provided is a computer-readable storage medium as defined by claim 14.

The operation program according may further cause the computer to function as the imaging failure image management apparatus that manages at least an imaging failure image among radiation images of a subject captured by the radiography apparatus.

The technology of the present disclosure relates to a radiography system comprising any one of the imaging failure image management apparatuses described above, and the radiography apparatus.

According to the technology of the present disclosure, it is possible to suppress the decrease in the determination accuracy of the imaging failure determination device mounted on the radiography apparatus.

### BRIEF DESCRIPTION OF THE DRAWINGS

Fig. 1 is a diagram showing an entire configuration of a radiography system.
Fig. 2 is a diagram showing an example of an imaging failure image.
Fig. 3 is a diagram showing a configuration of a console.
Fig. 4 is a diagram showing an imaging failure determination device.
Fig. 5 is a diagram showing classification of imaging failure determination results.
Fig. 6 is a diagram showing an example of an unapproved data set.
Fig. 7 is a hardware configuration diagram of an imaging failure image management apparatus.
Fig. 8 is a functional block diagram of the imaging failure image management apparatus.
Fig. 9 is a diagram showing a conference screen.
Fig. 10 is a diagram showing a conference screen in a case in which a reason for an imaging failure is corrected.
Fig. 11 is a diagram showing a conference screen on which an approved data set is displayed.
Fig. 12 is a diagram showing a conference screen in a case in which the reason for the imaging failure is not corrected.
Fig. 13 is a diagram showing another example of the conference screen on which the approved data set is displayed.
Fig. 14 is a diagram showing a training unit.
Fig. 15 is a diagram showing test processing.
Fig. 16 is a diagram showing an update notification.
Fig. 17 is a flowchart of an entire system in a case in which the imaging failure determination device is updated.
Fig. 18 is a flowchart showing a procedure for generating and outputting the unapproved data set.
Fig. 19 is a flowchart showing a procedure for generating and outputting the approved data set.
Fig. 20 is a flowchart showing a procedure of training processing.
Fig. 21 is an explanatory diagram showing a case in which a review target is narrowed down.

### DESCRIPTION OF THE PREFERRED EMBODIMENTS

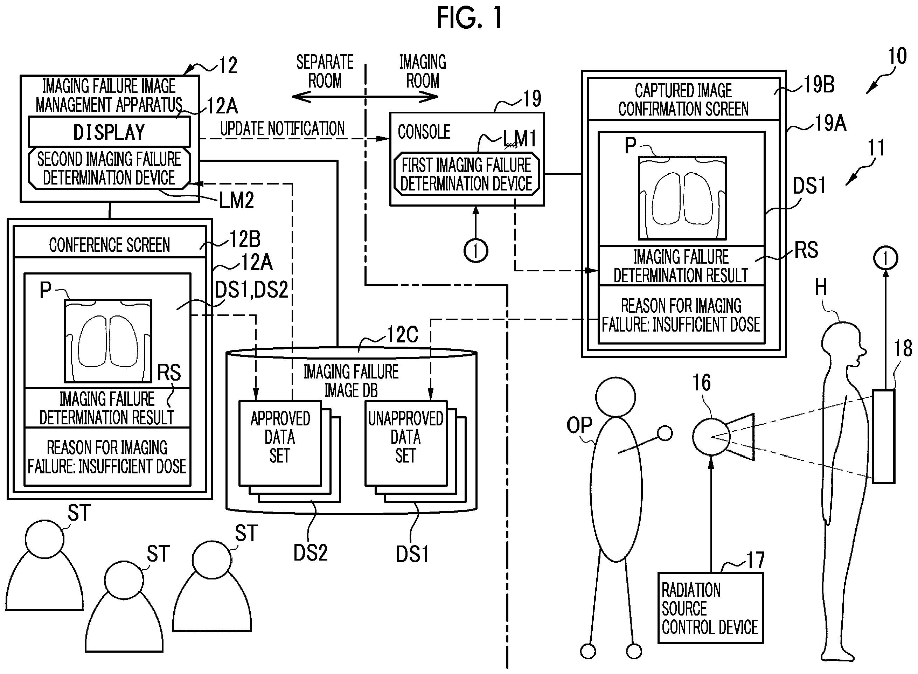

A radiography system 10 shown in Fig. 1 comprises a radiography apparatus 11 and an imaging failure image management apparatus 12. The radiography system 10 is installed in, for example, a medical facility. In the present example, the radiography system 10 has an imaging failure determination device update function of updating an imaging failure determination device mounted on the radiography apparatus 11. The imaging failure determination device is a machine learning model that is configured by a machine learning model and performs an imaging failure determination for a radiation image P captured by the radiography apparatus 11. In a case in which the radiation image P is input as input data, the imaging failure determination device outputs an imaging failure determination result RS as output data. The imaging failure determination result RS is an example of a "determination result" according to the technology of the present disclosure.

A first imaging failure determination device LM1 as an imaging failure determination device is mounted on the radiography apparatus 11 of the present example. Since the first imaging failure determination device LM1 is mounted, the radiography apparatus 11 has a function of presenting the imaging failure determination result RS for the captured radiation image P to an operator OP immediately after radiography is performed. On the other hand, the imaging failure image management apparatus 12 comprises a second imaging failure determination device LM2 as an imaging failure determination device. The second imaging failure determination device LM2 is an imaging failure determination device that can be substituted with the first imaging failure determination device LM1, but is not mounted on the radiography apparatus 11. The imaging failure image management apparatus 12 has a training function of training the second imaging failure determination device LM2. The radiography system 10 can update the first imaging failure determination device LM1 mounted on the radiography apparatus 11 by the second imaging failure determination device LM2 trained in the imaging failure image management apparatus 12.

As is well known, the radiography apparatus 11 obtains the radiation image P of a test subject H by imaging the test subject H using radiation, such as X-rays. The test subject H is an example of a subject. The radiography apparatus 11 is installed in, for example, an imaging room and the like. The radiography apparatus 11 comprises a radiation source 16, a radiation source control device 17, a radiation image detection device 18, and a console 19.

The radiation source 16 emits the radiation. The radiation source control device 17 controls the radiation source 16. An operation panel (not shown) is provided in the radiation source control device 17. An irradiation condition of the radiation is set by the operator OP operating the operation panel. The irradiation condition of the radiation includes a tube voltage (unit: kv) applied to the radiation source 16, a tube current (unit: mA), and an irradiation time (unit: mS) of the radiation. In addition, the radiation source 16 is provided with an irradiation field limiter that limits an irradiation field of the radiation. The irradiation field is adjusted by a manual operation or in an electrical manner. In addition, the radiation source 16 is attached to a moving mechanism (not shown), and a relative distance, height, irradiation direction, and the like with the test subject H can be adjusted.

The radiation image detection device 18 detects the radiation image P of the test subject H by receiving the radiation that is emitted from the radiation source 16 and transmitted through the test subject H. As the radiation image detection device 18, a flat panel detector and the like in which pixels for converting the radiation into an electric signal are two-dimensionally arranged is used. The radiation image detection device 18 includes a direct conversion type and an indirect conversion type as a method of converting the radiation into the electric signal. In addition, examples of a form thereof include a stationary type, which can be attached to a decubitus imaging table and a standing imaging table, and a portable type electronic cassette. The electronic cassette can be used alone, and can be used in combination with the imaging table. In the stationary type as well, the height and the like of the radiation image detection device 18 can be adjusted.

In a case in which the radiography is performed by using the radiography apparatus 11, the operator OP, such as a radiologist, performs positioning of the test subject H with respect to the radiography apparatus 11 by adjusting a relative positional relationship between the radiation source 16 and the radiation image detection device 18, and the test subject H. In addition, the operator OP sets the irradiation condition and the like according to an imaging part, a body thickness of the test subject H, and the like.

The console 19 acquires the radiation image P detected by the radiation image detection device 18, and displays the radiation image P on a display 19A. For example, a captured image confirmation screen 19B is displayed on the display 19A. The first imaging failure determination device LM1 is mounted on the console 19, and the imaging failure determination using the first imaging failure determination device LM1 is executed on the console 19. In addition to the radiation image P, the imaging failure determination result RS by the first imaging failure determination device LM1 can be displayed on the captured image confirmation screen 19B.

Reasons for an imaging failure, which is the basis for the radiation image P to be regarded as an imaging failure image, include inappropriate positioning of the imaging part due to a positioning mistake, insufficient dose of the radiation, and the like. The operator OP sets the positioning of the test subject H and the irradiation condition in a case of the radiography. Since the radiography apparatus 11 has an imaging failure determination function, the operator OP can confirm the presence or absence of a possibility that the radiation image P corresponds to the imaging failure image immediately after the radiation image P is captured. Then, in a case in which re-imaging is required, the operator OP can perform the correction of the positioning, the irradiation condition, and the like by confirming the imaging failure determination result RS.

The imaging failure determination is not limited to the determination as to whether or not the radiation image P is the imaging failure image, and need only include at least a determination of the presence or absence of a possibility that the radiation image P corresponds to the imaging failure image. The determination of the presence or absence of the possibility of the correspondence to the imaging failure image include at least one of a determination of the presence or absence of the reason for the imaging failure which is the basis being regarded as the imaging failure image, the presence or absence of the reason for the imaging failure having the possibility of the correspondence, a determination of a content of the reason for the imaging failure, or a determination as to whether or not the re-imaging is required. Then, the imaging failure determination result RS indicating that there is the possibility of the correspondence to the imaging failure image includes the imaging failure determination results RS, such as "it is the imaging failure image", "there is a possibility of the correspondence to the imaging failure image", "the presence or absence of the reason for the imaging failure and the content thereof", and "re-imaging required".

In the imaging failure determination by the first imaging failure determination device LM1 of the present example, the presence or absence of the reason for the imaging failure for the radiation image P and the content thereof are determined. In the present example, as the imaging failure determination result RS, the reason for the imaging failure, such as "insufficient dose", is output only in a case in which there is the reason for the imaging failure, and nothing is output in a case in which there is no reason for the imaging failure. That is, in the imaging failure determination result RS, the presence or absence of the reason for the imaging failure is indicated depending on whether or not a specific reason for the imaging failure is included. Of course, in addition to the specific reason for the imaging failure, the imaging failure determination result RS, such as "presence or absence of the reason for the imaging failure" and "appropriate or inappropriate", may be output.

The console 19 has the imaging failure determination function using the first imaging failure determination device LM1, and can also receive the input of the imaging failure determination result RS by the operator OP in a case in which the operator OP determines the imaging failure determination result RS of the first imaging failure determination device LM1 is incorrect. Here, the imaging failure determination result RS by the first imaging failure determination device LM1 is referred to as a primary determination result RS1, and the imaging failure determination result RS by the operator OP is referred to as a secondary determination result RS2.

The console 19 transmits a data set DS1 including the radiation image P and the imaging failure determination result RS to the imaging failure image management apparatus 12. In the present example, a conference on the data set DS1 is held by using the imaging failure image management apparatus 12, as described below. The conference is a meeting in which a user reviews the appropriateness of the imaging failure determination result RS included in the data set DS1. The user of the present example is a medical staff ST, such as a doctor or a technician in the medical facility in which the imaging failure image management apparatus 12 is installed. In the conference, in a case in which the imaging failure determination result RS of the data set DS1 is approved by the user, the data set DS1 is approved. Here, in order to distinguish the data set according to whether or not the approval by the user is made, an unapproved data set is referred to as an unapproved data set DS1, and an approved data set is referred to as an approved data set DS2. The console 19 transmits the unapproved data set DS1 to an imaging failure image data base (DB) 12C of the imaging failure image management apparatus 12.

The imaging failure image management apparatus 12 manages at least the imaging failure image among the radiation images P captured by the radiography apparatus 11. The imaging failure image refers to a radiation image in which the imaging has failed, and refers to an image that is not used for diagnosis. A determination as to whether or not the imaging has failed is generally based on the determination of the operator OP. A general imaging failure image management apparatus collects such imaging failure images, and manages the collected imaging failure images. The imaging failure image management apparatus 12 of the present example also manages the radiation image P having a possibility of the imaging failure, in addition to the imaging failure image. The reason is that, as will be described below, in addition to the imaging failure image, the radiation image P having the possibility of the imaging failure is also used as training data for training the second imaging failure determination device LM2. Of course, the imaging failure image management apparatus 12 can also manage the radiation image P other than the imaging failure image, such as a re-captured image PR associated with the imaging failure image, in addition to the imaging failure image. The re-captured image PR associated with the imaging failure image is the radiation image P that is re-captured in a case in which the radiation image P of the first imaging is the imaging failure image.

The imaging failure image management apparatus 12 has, for example, a function of managing the imaging failure image generated in the medical facility to execute statistical processing of the imaging failure image, the reason for the imaging failure, and the like. The statistical processing is, for example, processing of calculating statistical data, such as the number of cases of the imaging failure images, the number of cases of the reasons for the imaging failure for each content, and the number of cases of the imaging failure images for each imaging technique described below. The imaging failure image management apparatus 12 is installed in, for example, a room separate from the imaging room.

The imaging failure image management apparatus 12 has a conference function in addition to such functions. The conference function is, for example, a function used in the conference in which a plurality of medical staffs ST exchange opinions on the imaging failure image. In the conference, the appropriateness of the imaging failure determination result RS included in the unapproved data set DS1 that is a review target is verified through the exchange of the opinions of the plurality of medical staffs ST. For example, with respect to the imaging failure determination result RS, it is verified whether the determination that there is the reason for the imaging failure or no reason for the imaging failure in the radiation image P is appropriate, or whether a specific content of the reason for the imaging failure is appropriate. In the conference, in a case in which it is determined that the imaging failure determination result RS of the unapproved data set DS1 is appropriate, the imaging failure determination result RS is approved as it is without being corrected, and in a case in which it is determined that the imaging failure determination result RS is not appropriate, the imaging failure determination result RS is corrected as required, and the corrected imaging failure determination result RS is approved.

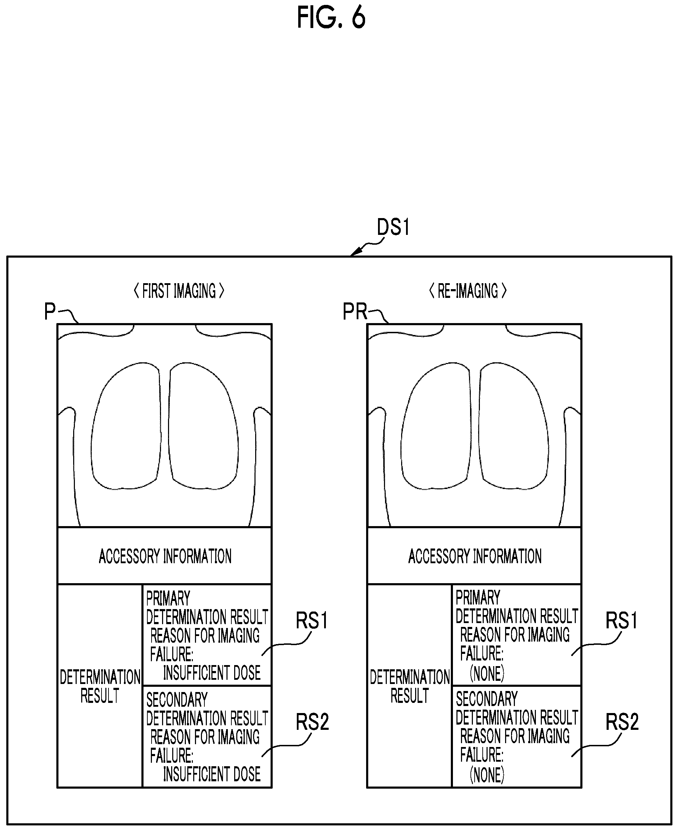

In addition, in some cases, the unapproved data set DS1 also includes the re-captured image PR (see Fig. 6 and the like). In such a case, the same verification is performed for the re-captured image PR. Through such a conference, the appropriateness of the imaging failure determination result RS is verified, and the verified imaging failure determination result RS is approved.

In the conference function of the imaging failure image management apparatus 12, in addition to displaying the unapproved data set DS1, which is the review target, on a display screen, such as the conference screen 12B of a display 12A, as described below, the correction of the imaging failure determination result RS included in the unapproved data set DS1, recording of approval information indicating that the approval is made for the uncorrected or corrected imaging failure determination result RS for which the appropriateness is verified, and the like can be performed.

The purpose of the conference, for example, is to educate an inexperienced medical staff ST. The medical staff ST who participates in the conference includes an experienced doctor or technician in a leading position. Since the appropriateness of the imaging failure determination result RS is verified by these plurality of medical staffs ST, it is considered that the reliability of the imaging failure determination result RS of the approved data set DS2 that has undergone the review of the conference is higher than the independent determination of the operator OP.

In a case in which the unapproved data set DS1 is approved, the imaging failure image management apparatus 12 stores the unapproved data set DS1 in the imaging failure image DB 12C as the approved data set DS2. In addition, the imaging failure image management apparatus 12 has a function of training the second imaging failure determination device LM2 that can be substituted with the first imaging failure determination device LM1. The imaging failure image management apparatus 12 additionally trains the second imaging failure determination device LM2 by using the approved data set DS2. In the present example, the second imaging failure determination device LM2 immediately before the training processing of the additional training is executed is the same as the first imaging failure determination device LM1. The additional training refers to training that is additionally performed in a process of actual operation after the second imaging failure determination device LM2 is generated by the programming and the training processing. In a case in which the additional training is executed, the first imaging failure determination device LM1 is updated by the second imaging failure determination device LM2 that is additionally trained. As described above, the radiography system 10 has a function of updating the first imaging failure determination device LM1 mounted on the radiography apparatus 11 by the radiography apparatus 11 and the imaging failure image management apparatus 12.

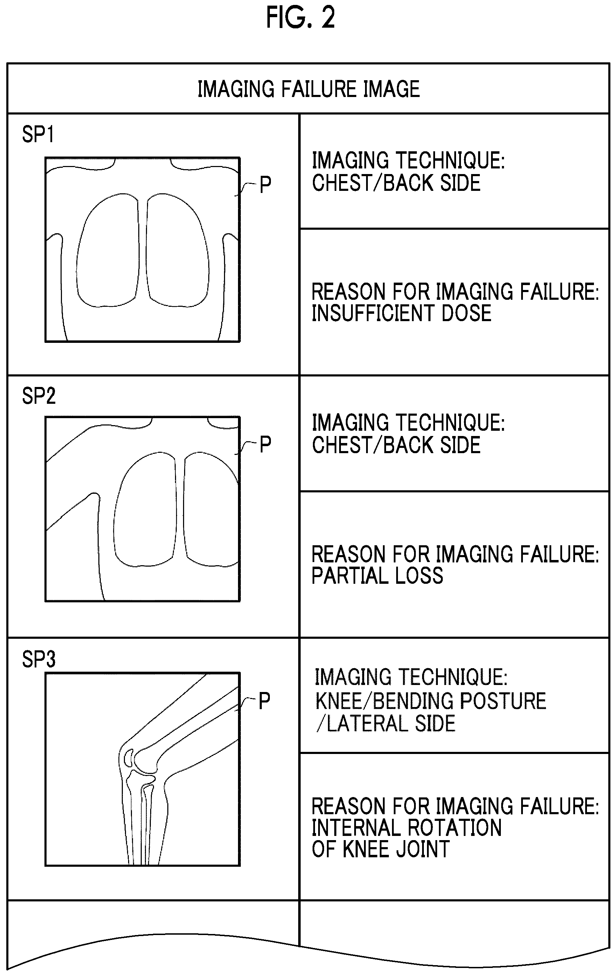

Fig. 2 shows samples SP1 to SP3 of the imaging failure image. The sample SP1 is the radiation image P in which the imaging technique is "chest/back side", that is, the radiation image P captured from the back side with the chest as the imaging part. The reason for the imaging failure of the sample SP1 is the insufficient dose of the radiation. The insufficient dose is mainly caused by a mistake in setting the irradiation condition. The imaging technique is an imaging method defined by at least a combination of the imaging part and an imaging direction. In an imaging order, the doctor designates the imaging technique including the imaging part and the imaging direction according to a diagnostic purpose of the test subject H.

The sample SP2 is the radiation image P in which the imaging technique is "chest/back side" that is the same as the sample SP1. The reason for the imaging failure of the sample SP2 is a partial loss of the imaging part. In the present example, a left chest is partially lost. The partial loss is mainly caused by the positioning mistake. The sample SP3 is the radiation image P in which the imaging technique is "knee/bending posture/lateral side", that is, the radiation image P captured from the lateral side with the knee as the imaging part in a state in which the knee is bent. The reason for the imaging failure of the sample SP3 is an internal rotation of a knee joint. Since a part of interest of the knee joint differs depending on the diagnostic purpose, how to bend the knee joint is important in the positioning. Therefore, the internal rotation or an external rotation of the knee joint may be the reason for the imaging failure depending on the diagnostic purpose.

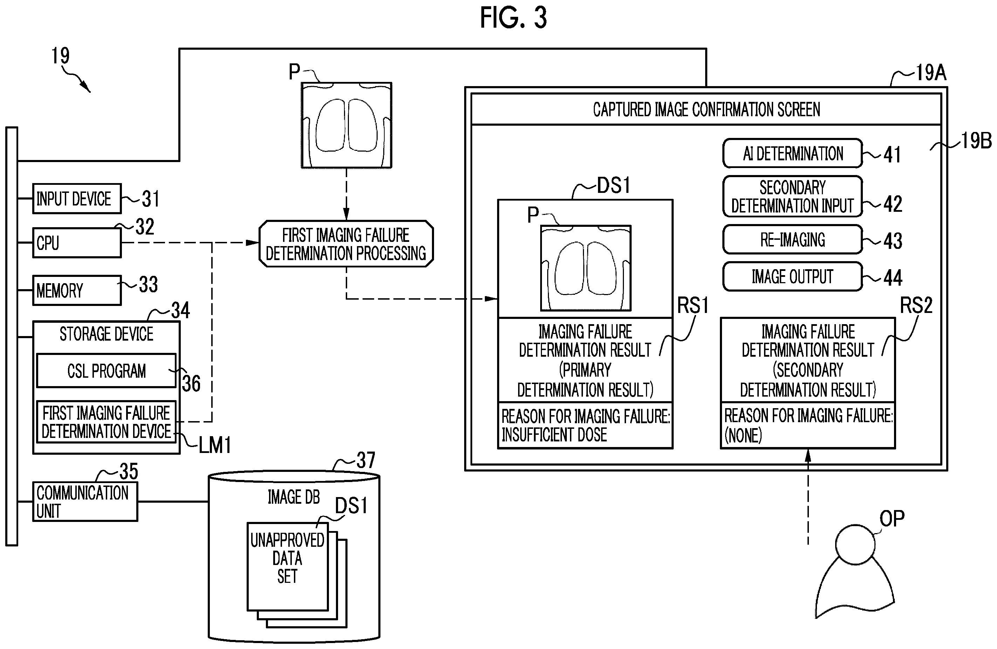

Hereinafter, a configuration and a function of the console 19 of the radiography apparatus 11 will be described in more detail with reference to Figs. 3 to 5.

The console 19 is configured by installing a console program (referred to as a CSL program in the figure) 36 based on a computer, such as a personal computer or a workstation. The console 19 comprises a display 19A, an input device 31, a central processing unit (CPU) 32, a storage device 34, a memory 33, and a communication unit 35. These components are connected to each other via a data bus.

The display 19A is a display unit that displays various operation screens provided with an operation function by a graphical user interface (GUI), the radiation image P, and the like. The input device 31 is an input operation unit including a touch panel, a keyboard, or the like.

The storage device 34 is, for example, a hard disk drive (HDD), a solid state drive (SSD), or the like, and is built in the console 19 or externally connected to the console 19. A control program, such as an operating system, various application programs, and various types of data associated with these programs are stored in the storage device 34.

In addition, the storage device 34 stores the console program 36 for causing the computer to function as the console 19, and the first imaging failure determination device LM1. In addition, the storage device 34 stores the radiation image P received from the radiation image detection device 18.

The memory 33 is a work memory for the CPU 32 to execute the processing. The CPU 32 loads the console program 36 stored in the storage device 34 into the memory 33, and executes the processing according to the console program 36 to control the respective units of the console 19 in an integrated manner. The communication unit 35 transmits and receives various types of data, such as the radiation image P, to and from the radiation image detection device 18. In addition, the communication unit 35 performs communication with the radiation source control device 17 and the imaging failure image management apparatus 12. The communication unit 35 performs communication via a network, such as a local area network (LAN).

Further, an image DB 37 is, for example, a storage device of a picture archiving and communication system (PACS). The unapproved data set DS1 is also transmitted to the PACS in addition to the imaging failure image management apparatus 12, and is stored in the image DB 37.

The captured image confirmation screen 19B is an example of a display screen displayed on the display 19A of the console 19, and is a screen for the operator OP to confirm the radiation image P for which the radiography is performed. The operator OP confirms the radiation image P on the captured image confirmation screen 19B, and determines whether or not the radiation image P is the imaging failure image, whether or not there is the possibility of the correspondence to the imaging failure image, what is the specific content of the reason for the imaging failure, and whether or not the re-imaging is required. The imaging failure determination function provided in the console 19 supports the work of the operator OP.

The captured image confirmation screen 19B is provided with operation buttons, such as an artificial intelligence (AI) determination button 41, a secondary determination input button 42, a re-imaging button 43, and an image output button 44. The AI determination button 41 is an operation button for executing first imaging failure determination processing using the first imaging failure determination device LM1.

In a case in which the AI determination button 41 is operated, the CPU 32 executes the first imaging failure determination processing using the first imaging failure determination device LM1.

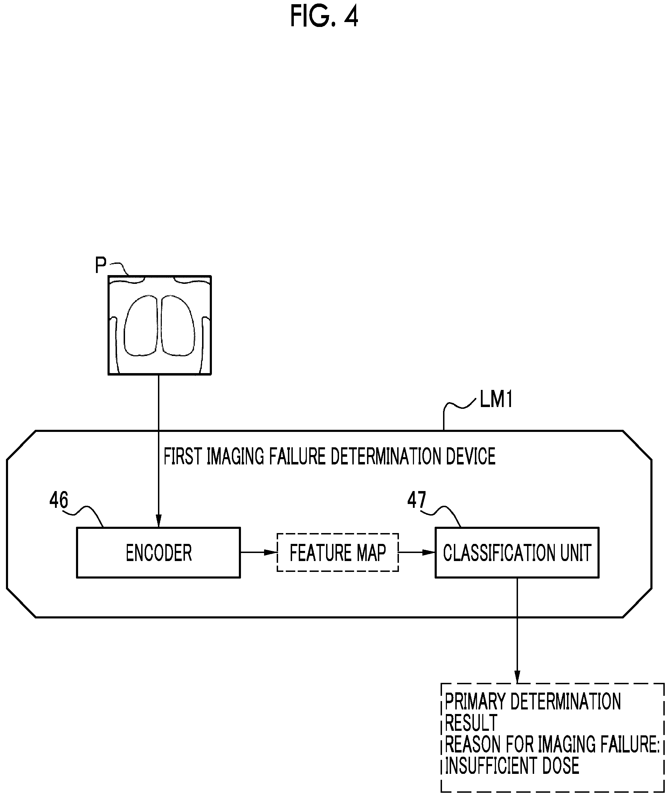

As shown in Fig. 4, as the first imaging failure determination device LM1, for example, a convolutional neural network (CNN) suitable for an image analysis is used. The first imaging failure determination device LM1 includes, for example, an encoder 46 and a classification unit 47.

The encoder 46 is configured by using the CNN, and executes convolution processing and pooling processing for the input radiation image P to extract a plurality of types of feature maps representing the features of the radiation image P. As is well known, in the CNN, the convolution is spatial filtering processing using a plurality of types of filters having a size of, for example, 3 × 3. In a case of the 3 × 3 filter, a filter coefficient is allocated to each of 9 cells. In the convolution, the cell of the center of the filter is aligned with a pixel of interest of the radiation image P, and the product sum of the pixel values of a total of 9 pixels of the pixel of interest and 8 pixels surrounding the pixel of interest is output. The output product sum represents a feature amount of a region of interest to which the filter is applied. Then, for example, by applying the filter to all the pixels of the radiation image P while shifting the pixel of interest one by one, the feature map having the feature amount equivalent to the number of pixels of the radiation image P is output. The plurality of types of feature maps are output by applying a plurality of types of filters having different filter coefficients. The number of feature maps according to the number of filters is also referred to as the number of channels.

Such convolution is repeated while reducing a size of the radiation image P. The processing of reducing the radiation image P as the pooling processing. The pooling processing is performed by executing thinning-out processing, averaging processing, and the like of adjacent pixels. By the pooling processing, the size of the radiation image P is gradually reduced to 1/2, 1/4, and 1/8. The feature maps of the plurality of channels are output for each size of the radiation image P. In a case in which the size of the radiation image P is large, detailed morphological features of the subject are visualized in the radiation image P. However, in a case in which the size of the radiation image P is small (that is, in a case in which a resolution is low), detailed morphological features of the subject are discarded from the radiation image P, and only rough morphological features of the subject are visualized in the radiation image P. Therefore, the feature map in a case in which the radiation image P is large represents the microscopic features of the subject visualized in the radiation image P, and the feature map in a case in which the size is small represents the macroscopic features of the subject visualized in the radiation image P. The encoder 46 performs such convolution processing and pooling processing on the radiation image P to extract the feature maps of the plurality of channels representing the macroscopic and microscopic features of the radiation image P.

The first imaging failure determination device LM1 is, for example, a classification model that derives one reason for the imaging failure (including a case in which there is no reason for the imaging failure) having the highest possibility as the reason for the imaging failure indicated by the radiation image P from among a plurality of reasons for the imaging failure. For this reason, the classification unit 47 is provided as a configuration for deriving one reason for the imaging failure based on the feature amount of the radiation image P extracted by the encoder 46. The classification unit 47 comprises, for example, a plurality of perceptrons having one output node for a plurality of input nodes. In addition, weights indicating the importance of the plurality of input nodes are allocated to the perceptrons. In each perceptron, the product sum, which is the sum of the values obtained by multiplying each of input values input to the plurality of input nodes by the weight, is output from the output node as an output value. Such a perceptron is formulated by, for example, an activation function, such as a sigmoid function.

In the classification unit 47, a multi-layered neural network having a plurality of interlayers between an input layer and an output layer is formed by connecting the output and the input of the plurality of perceptrons. As a method of connecting the plurality of perceptrons between layers, for example, full connection in which all the output nodes of the previous layer are connected to one input node of the next layer is adopted.

All the feature amounts included in the feature map of the radiation image P are input to the input layer of the classification unit 47. The feature amount is input to the input node as the input value in the perceptron that configures each layer of the classification unit 47. Then, the product sum, which is the sum of values obtained by multiplying the feature amount by the weight of each input node, is output as the output value from the output node, and the output value is passed to the input node of the perceptron of the next layer. In the classification unit 47, the output layer of the final layer outputs a probability of each of a plurality of types of the reasons for the imaging failure by using a softmax function or the like based on the output values of the plurality of perceptrons. Then, one reason for the imaging failure having the highest possibility is derived based on this probability. Fig. 4 shows an example in which the reason for the imaging failure, that is, "insufficient dose" is derived, based on the radiation image P. The imaging failure determination result output by the first imaging failure determination device LM1 is the primary determination result.

The present example is an example, and the first imaging failure determination device LM1 may have another aspect as long as the first imaging failure determination device LM1 is the classification model can derive the reason for the imaging failure based on the radiation image P. In addition, in the present example, although only one first imaging failure determination device LM1 is used without distinguishing between the imaging techniques, the first imaging failure determination device LM1 may be prepared for each imaging technique. Since some of the reasons for the imaging failure differ for each imaging technique, the determination accuracy is improved by using the first imaging failure determination device LM1 for each imaging technique.

In Fig. 3, the primary determination result RS1 which is the imaging failure determination result RS output by the first imaging failure determination device LM1 is displayed on the captured image confirmation screen 19B.

The secondary determination input button 42 is an operation button for inputting the secondary determination result RS2 which is the imaging failure determination result RS by the operator OP. The operator OP can input the secondary determination result RS2 of the operator OP in a case in which the primary determination result RS1 is different from the determination of the operator OP. In a case in which the secondary determination input button 42 is operated, the secondary determination result RS2 can be input, and the operator OP operates the input device 31 to input the secondary determination result RS2. The example of Fig. 3 shows an example in which the primary determination result RS1 is the insufficient dose, whereas the secondary determination result RS2 of the operator OP is input as "no reason for the imaging failure".

The re-imaging button 43 is an operation button that is operated in a case in which the re-imaging is required. In a case in which the operator OP determines that the re-imaging is required, the operator OP operates the re-imaging button 43 to perform the re-imaging. In a case in which the re-imaging button 43 is operated, for example, the radiation image P of the first imaging and the radiation image P of the re-imaging are associated with each other. The association is performed, for example, by an order identification data (ID) of the imaging order. As a result, it is possible to collectively treat the radiation image P of the first imaging and the radiation image P of the re-imaging.

The image output button 44 is a button for outputting the unapproved data set DS1 including the radiation image P and the imaging failure determination result RS to the image DB 37 and the imaging failure image DB 12C. In a case in which the secondary determination result RS2 is input, the imaging failure determination result RS of the unapproved data set DS1 includes both the primary determination result RS1 and the secondary determination result RS2. In a case in which the secondary determination result RS2 is not input, only the primary determination result RS1 is included.

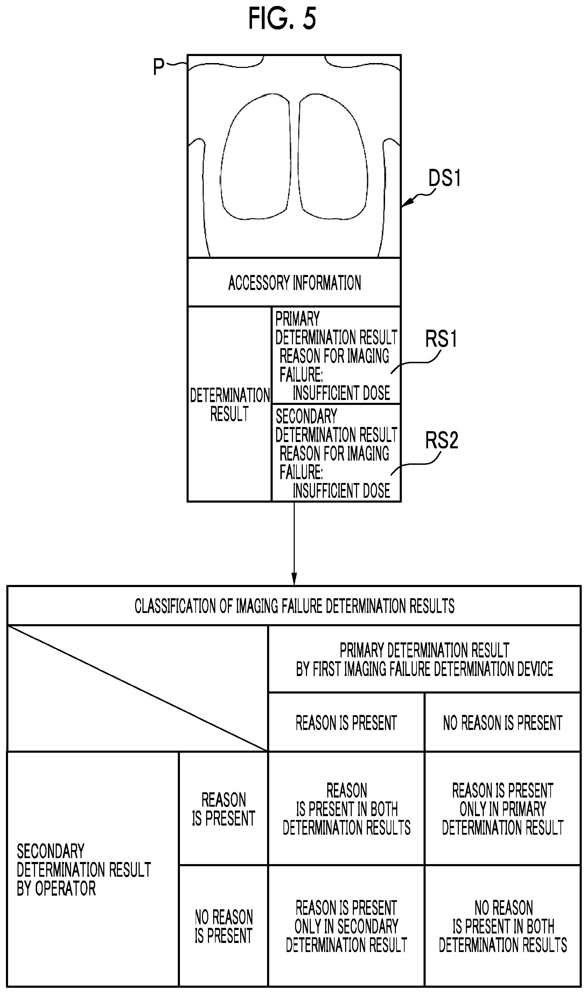

As shown in Fig. 5, the unapproved data set DS1 includes accessory information incidental to the radiation image P in addition to the imaging failure determination result RS. The accessory information is data, such as a name, age, gender, the order ID, and the imaging technique of the test subject H. In the example of Fig. 5, both the primary determination result RS1 and the secondary determination result RS2 are included as the imaging failure determination result RS.

The type of the imaging failure determination result RS included in the unapproved data set DS1 can be roughly classified into four types shown in Fig. 5 by a combination of the presence or absence of the reason for the imaging failure of the primary determination result RS1 and the presence or absence of the reason for the imaging failure of the secondary determination result RS2. That is, there are four types, which are a case in which the reason is present in both the primary determination result RS1 and the secondary determination result RS2, a case in which the reason for the imaging failure is present only in the primary determination result RS1, a case in which the reason is present only in the secondary determination result RS2, and a case in which no reason is present in both the primary determination result RS1 and the secondary determination result RS2.

Of course, since the secondary determination result RS2 is information that is input as required in the determination of the operator OP, more specifically, in addition to the four types shown in Fig. 5, the secondary determination result RS2 may not be input. In addition, even in a case in which the reason for the imaging failure is present in both the primary determination result RS1 and the secondary determination result RS2, the reason for the imaging failure is the same between the primary determination result RS1 and the secondary determination result RS2. For distinction, in the unapproved data set DS1, in addition to the presence or absence of the reason for the imaging failure of the primary determination result RS1 and the specific content ("insufficient dose" in the example of Fig. 5) of the reason for the imaging failure, in a case in which the secondary determination result RS2 is present, the presence or absence of the reason for the imaging failure of the secondary determination result RS2 and the specific content ("insufficient dose" in the example of Fig. 5) of the reason for the imaging failure are recorded.

In addition, as shown in Fig. 6, in a case in which the re-imaging is performed, the re-captured image PR is included in the unapproved data set DS1. For the re-captured image PR as well, the primary determination result RS1 and the secondary determination result RS2 are recorded, respectively. The re-captured image PR is an image determined by the operator OP that there is no reason for the imaging failure. Therefore, in the imaging failure determination result RS of the re-captured image PR, at least the secondary determination result RS2 has no reason for the imaging failure. The re-captured image PR of Fig. 6 is an example in which both the primary determination result RS1 and the secondary determination result RS2 have no reason for the imaging failure.

In a case in which the unapproved data set DS1 is generated by performing the radiography and the imaging failure determination using the radiography apparatus 11, the console 19 notifies the imaging failure image management apparatus 12 that the unapproved data set DS1 is generated. The imaging failure image management apparatus 12 can recognize that the unapproved data set DS1 is generated, by this notification.

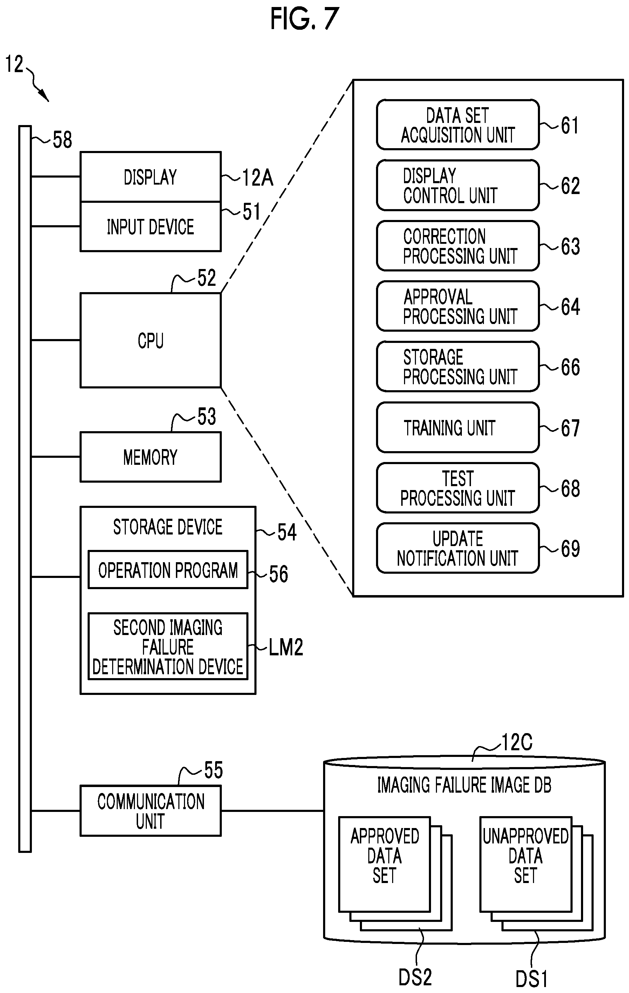

Hereinafter, a configuration and a function of the imaging failure image management apparatus 12 will be described in more detail with reference to Figs. 7 to 16. As shown in Fig. 7, the imaging failure image management apparatus 12 is configured by installing an operation program 56 based on a computer such as a personal computer or a workstation, similarly to the console 19. The operation program 56 is an example of an operation program of the imaging failure image management apparatus 12 according to the technology of the present disclosure. The imaging failure image management apparatus 12 comprises a display 12A, an input device 51, a CPU 52, a storage device 54, a memory 53, and a communication unit 55. These components are connected to each other via a data bus 58.

The display 12A is a display unit that displays various operation screens provided with an operation function using a GUI, the radiation image P, and the like. The input device 51 is an input operation unit including a touch panel, a keyboard, or the like.

The storage device 54 is, for example, an HDD, an SSD, or the like, and is built in the imaging failure image management apparatus 12 or externally connected to the imaging failure image management apparatus 12. A control program, such as an operating system, various application programs, and various types of data associated with these programs are stored in the storage device 54.

In addition, the storage device 54 stores the operation program 56 for causing the computer to function as the imaging failure image management apparatus 12, and the second imaging failure determination device LM2. In addition, the storage device 54 also transitorily stores the unapproved data set DS1 acquired from the console 19 or the image DB 37.

The memory 53 is a work memory for the CPU 52 to execute the processing. The CPU 52 loads the operation program 56 stored in the storage device 54 into the memory 53, and executes the processing according to the operation program 56 to control the respective units of the imaging failure image management apparatus 12 in an integrated manner. The communication unit 55 performs communication with the console 19 via a network, such as the LAN.

In addition, the imaging failure image DB 12C is an external storage device of the imaging failure image management apparatus 12. As described above, the imaging failure image DB 12C stores the unapproved data set DS1 and the approved data set DS2.

By executing the operation program 56, the CPU 52 functions as various processing units, such as a data set acquisition unit 61, a display control unit 62, a correction processing unit 63, an approval processing unit 64, a storage processing unit 66, a training unit 67, a test processing unit 68, and an update notification unit 69.

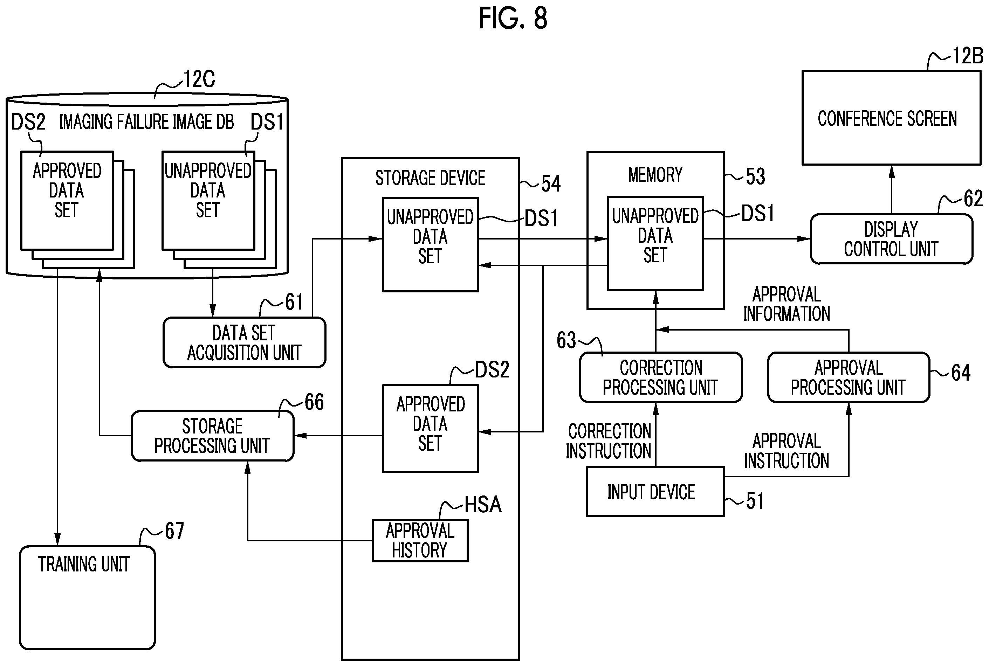

As shown in Fig. 8, the data set acquisition unit 61 acquires the unapproved data set DS1 from the imaging failure image DB 12C, and stores the acquired unapproved data set DS1 in the storage device 54. As described above, the data set acquisition unit 61 executes data set acquisition processing of acquiring the unapproved data set DS1 including the radiation image P input to the first imaging failure determination device LM1 mounted on the radiography apparatus 11 and the imaging failure determination result RS output from the first imaging failure determination device LM1. In the present example, the data set acquisition unit 61 acquires the unapproved data set DS1 from the imaging failure image DB 12C which is the external storage of the imaging failure image management apparatus 12, but may access the image DB 37 of the PACS to acquire the image DB 37. Further, in a case in which the unapproved data set DS1 is stored in a shared folder of the storage device 34 of the console 19, the data set acquisition unit 61 may access the storage device 34 of the console 19 to acquire the data set from the shared folder.

The display control unit 62 performs control of expanding screen data of the conference screen 12B including the unapproved data set DS1 to the memory 53, and displaying the conference screen 12B on the display 12A based on the screen data expanded in the memory 53. In this way, the display control unit 62 executes display control processing of performing control of displaying the unapproved data set DS1 on the display 12A.

The correction processing unit 63 executes correction processing of performing the correction for the imaging failure determination result RS in a case in which a correction instruction for the imaging failure determination result RS of the unapproved data set DS1 displayed on the conference screen 12B is input by the user. The correction processing unit 63 updates the imaging failure determination result RS of the unapproved data set DS1 which is a correction target.

The approval processing unit 64 executes approval processing in a case in which an approval instruction by the user is input for the imaging failure determination result RS of the unapproved data set DS1 displayed on the conference screen 12B. The approval processing is processing of recording the approval information indicating that the imaging failure determination result RS is approved in association with the unapproved data set DS1 which is an approval target. The approval information is recorded as, for example, an approval history HSA. In a case in which the unapproved data set DS1 is approved, the unapproved data set DS1 is changed to the approved data set DS2. In the approval history HSA, for example, ID information of the approved data set DS2 and the approval date and time are recorded. The approval history HSA is stored in, for example, the imaging failure image DB 12C, similar to the approved data set DS2.

The approval history HSA has the meaning as information indicating that the unapproved data set DS1 is displayed on the conference screen 12B. In addition, in a case in which the unapproved data set DS1 is displayed on the conference screen 12B, it is considered that a possibility that the displayed unapproved data set DS1 is used as the review target in the conference is high. Therefore, the approval history HSA has the meaning as information indicating that the review by the medical staff ST who is the user of the imaging failure determination result RS of the unapproved data set DS1 is performed in the conference. As described above, the approval history HSA is an example of information indicating that the unapproved data set DS1 is displayed on the display screen or information indicating that the review is performed.

The storage processing unit 66 executes storage processing of storing the approved data set DS2 and the approval history HSA in the imaging failure image DB 12C in a case in which a save instruction is input from the user.

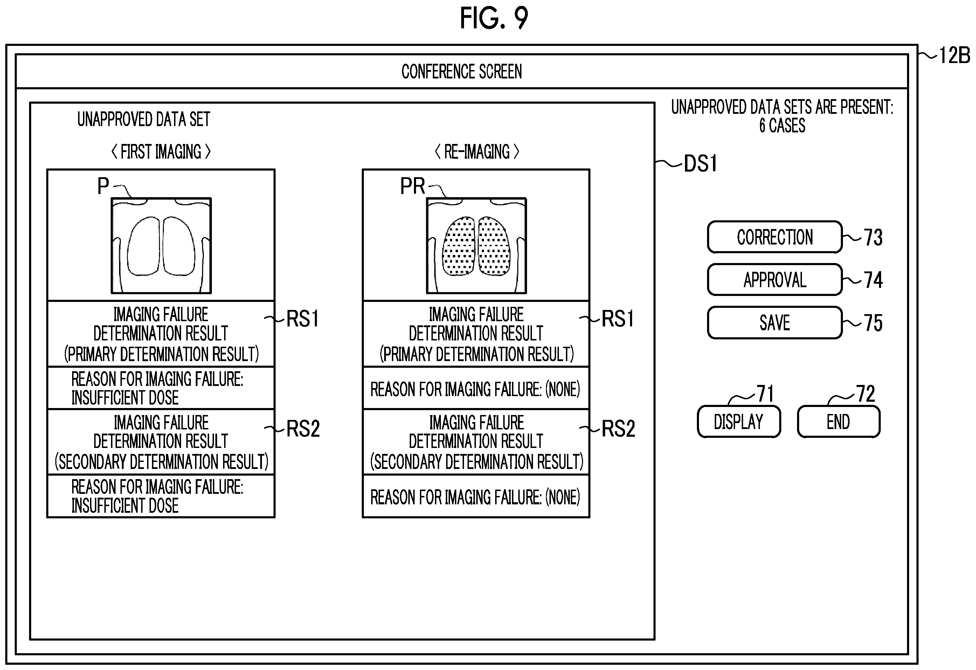

Fig. 9 shows an example of the conference screen 12B. The unapproved data set DS1 is displayed on the conference screen 12B. As an example, the unapproved data set DS1, which is the review target in the conference, is displayed on the conference screen 12B one case by one case. The radiation image P included in the unapproved data set DS1 and the imaging failure determination result RS are displayed on the conference screen 12B. The unapproved data set DS1 shown in Fig. 9 is an example in which both the radiation image P of the first imaging and the re-captured image PR are included, and the imaging failure determination results RS of both the radiation image P of the first imaging and the re-captured image PR is are included. Further, the imaging failure determination result RS of the example of Fig. 9 includes the primary determination result RS1 and the secondary determination result RS2.

In a case in which the unapproved data set DS1 includes both the primary determination result RS1 output by the first imaging failure determination device LM1 and the secondary determination result RS2 by the operator OP, the display control unit 62 displays the primary determination result RS1 and the secondary determination result RS2 in a distinguishable manner in the display control processing. As a result, it is possible to verify the appropriateness of each of the primary determination result RS1 and the secondary determination result RS2 in a distinguishable manner. As an example, the conference proceeds by allowing the plurality of medical staffs ST to exchange the opinions while viewing the conference screen 12B.

The conference screen 12B is provided with various operation buttons, such as a display button 71, an end button 72, a correction button 73, an approval button 74, and a save button 75. The display button 71 is an operation button for displaying the unapproved data set DS1. In a case in which the display button 71 is operated, a display instruction is input to the display control unit 62, and the unapproved data set DS1 acquired through the data set acquisition unit 61 is displayed on the conference screen 12B. The end button 72 is an operation button for closing the conference screen 12B. In a case in which the conference ends, the conference screen 12B is closed.

The correction button 73 is a button that is operated in a case in which the imaging failure determination result RS of the unapproved data set DS1 is corrected. In a case in which the correction button 73 is operated, the imaging failure determination result RS of the unapproved data set DS1 can be edited. The correction is performed by using the keyboard of the input device 51 and the like. The correction instruction by the correction button 73 is input to the correction processing unit 63, and the correction processing unit 63 corrects the imaging failure determination result RS according to the correction instruction.

The approval button 74 is a button operated in a case in which the imaging failure determination result RS of the unapproved data set DS1 is approved. In a case in which the approval button 74 is operated, the approval instruction is input to the approval processing unit 64, and the approval processing unit 64 executes the approval processing.

The save button 75 is a button for saving the unapproved data set DS1 as the approved data set DS2 in a case in which the unapproved data set DS1 is approved. In a case in which the save button 75 is operated, the save instruction is input to the storage processing unit 66. Based on the save instruction, the storage processing unit 66 executes storage processing of storing the approved data set DS2 in the imaging failure image DB 12C.

In addition, information indicating how many cases of the unapproved data sets DS1 are present in the imaging failure image DB 12C is displayed on the conference screen 12B. In the present example, information indicating that there are six cases of the unapproved data sets DS1 is displayed.

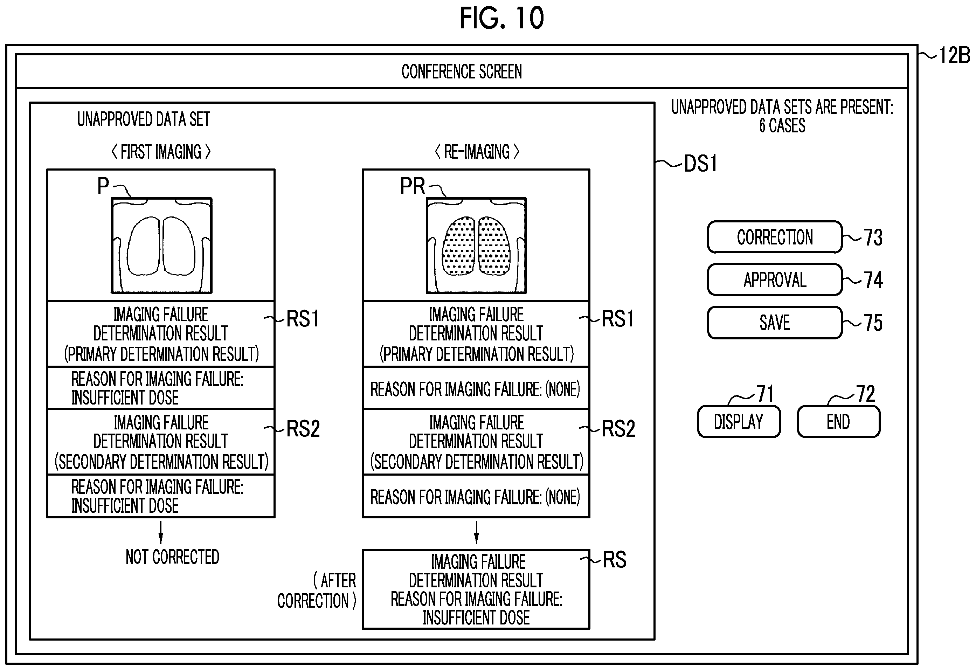

Fig. 10 shows an example in which the imaging failure determination result RS of the unapproved data set DS1 shown in Fig. 9 is corrected. In the example shown in Fig. 10, the imaging failure determination result RS of the re-captured image PR is corrected. As shown in Fig. 9, it is determined that the reason for the imaging failure, such as "insufficient dose", is present in both the primary determination result RS1 and the secondary determination result RS2 in the radiation image P of the first imaging, a result that there is no reason for the imaging failure in both the primary determination result RS1 and the secondary determination result RS2 is shown in the imaging failure determination result RS of the re-captured image PR. By reviewing the unapproved data set DS1 in the conference, as shown in Fig. 10 as an example, the imaging failure determination result RS of the re-captured image PR is corrected from "no reason for the imaging failure" to "insufficient dose". On the other hand, the imaging failure determination result RS of the radiation image P of the first imaging is determined to be appropriate, and the correction is not performed.

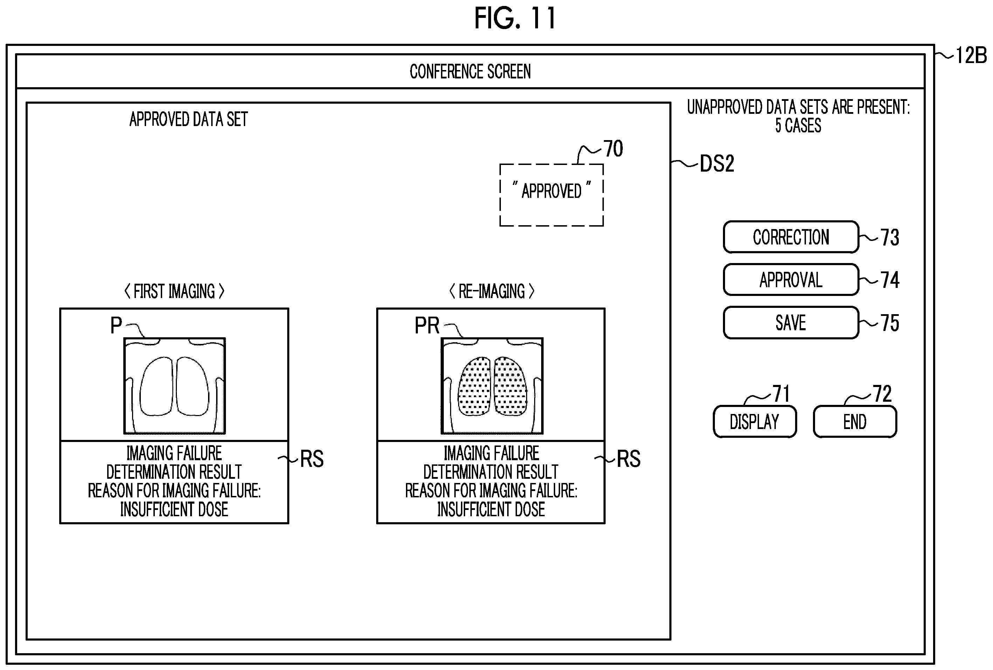

In a case in which the approval processing is performed on the unapproved data set DS1 shown in Fig. 10 by operating the approval button 74, as shown in Fig. 11, the unapproved data set DS1 shown in Fig. 10 is changed to the approved data set DS2. In the example of Fig. 11, a flag 70 indicating the approval is performed is displayed in the approved data set DS2 on the conference screen 12B. The flag 70 is, for example, a text "approved". As shown in Fig. 11, in the approved data set DS2, in each of the radiation image P of the first imaging and the re-captured image PR, one imaging failure determination result RS is recorded as a unified view of the plurality of medical staffs ST who are reviewers of the conference. In a case in which the save button 75 is operated, the approved data set DS2 is stored in the imaging failure image DB 12C. In a case in which one case of the approved data set DS2 is newly stored, the display of the remaining number of cases of unapproved data sets DS1 is reduced. The number of cases, which is six cases in Fig. 10, is changed to five cases in Fig. 11.

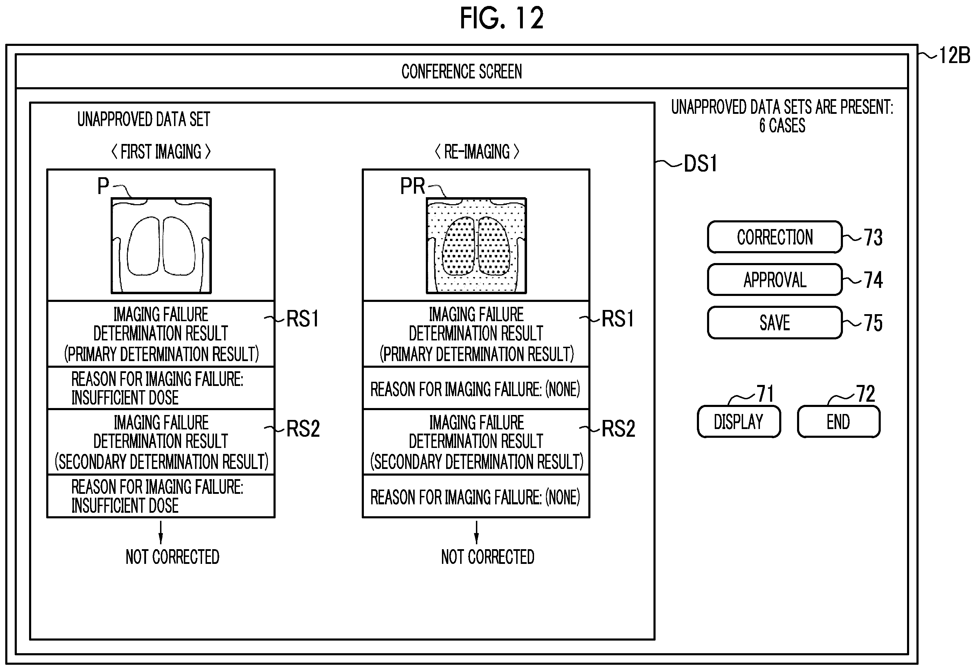

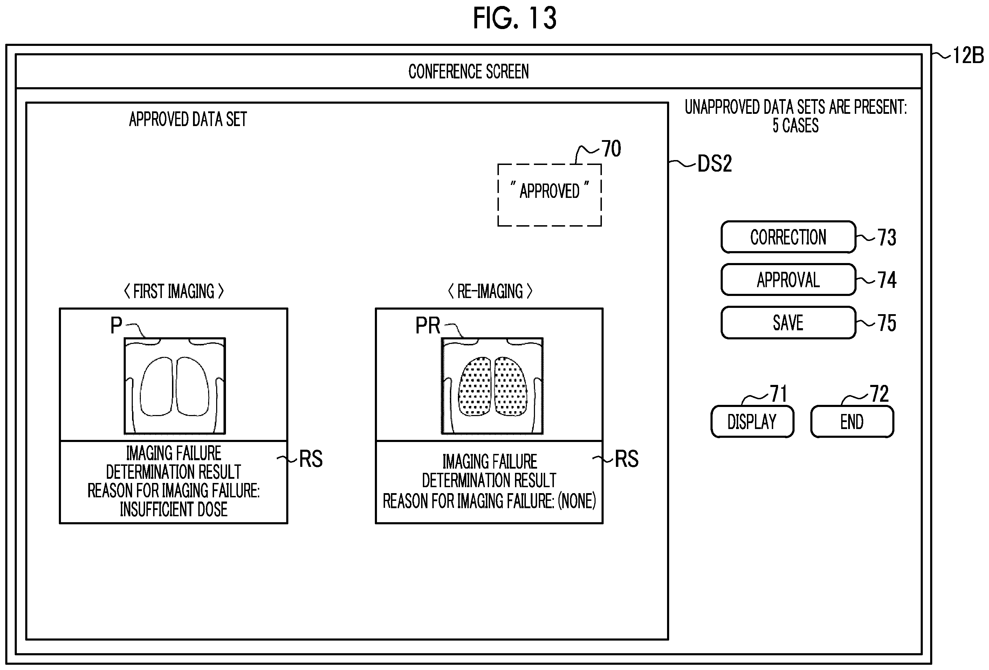

In the example of Figs. 12 and 13, a case is shown in which the imaging failure determination result RS of the unapproved data set DS1 shown in Fig. 12 is approved as it is without performing the correction, and is changed to the approved data set DS2 as shown in Fig. 13. Both the unapproved data set DS1 shown in Fig. 12 and the approved data set DS2 shown in Fig. 13 have the same imaging failure determination result RS. In this way, by approving the imaging failure determination result RS of the unapproved data set DS1 as it is, the imaging failure determination result RS of the approved data set DS2 is also obtained, in some cases.

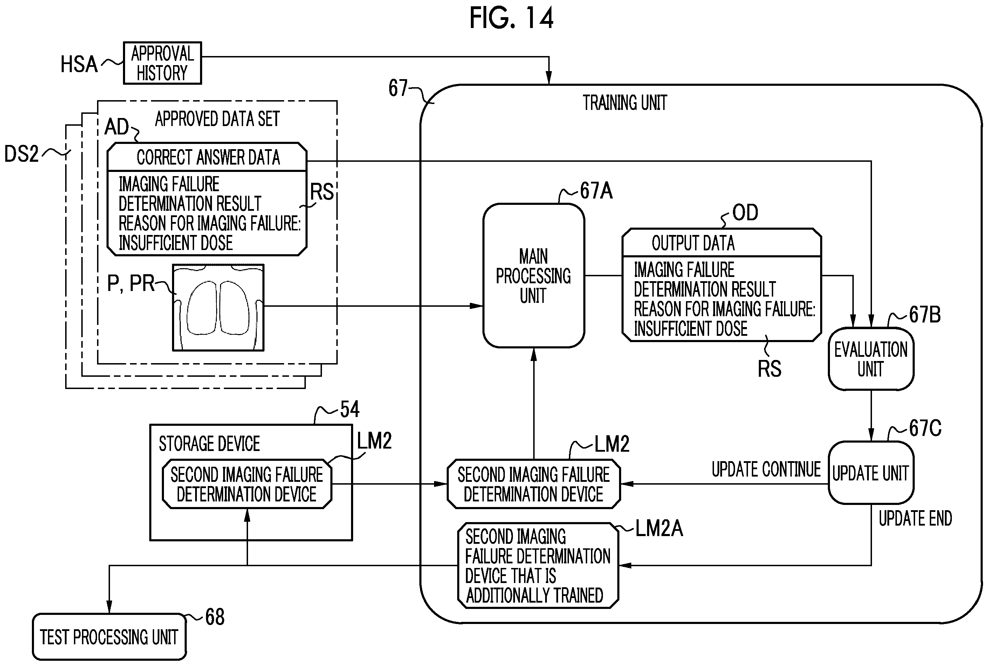

As shown in Fig. 14, the training unit 67 executes the training processing of training the second imaging failure determination device LM2 that can be substituted with the first imaging failure determination device LM1 and is not mounted on the radiography apparatus 11. The training processing executed by the training unit 67 is training processing of training the second imaging failure determination device LM2 by using the training data selected from the approved data set DS2 displayed on the display 12A of the imaging failure image management apparatus 12, that is, the approved data set DS2 stored in the imaging failure image DB 12C. In addition, the training processing executed by the training unit 67 is additional training for the second imaging failure determination device LM2. The training unit 67 selects the approved data set DS2 from the imaging failure image DB 12C as the training data based on the approval history HSA. The approved data set DS2 is not required to be selected as the training data as a whole, and a part thereof may be selected. A criterion for the selection as the training data from the approved data set DS2 can be appropriately set. In addition, the training data may be selected by the training unit 67, or may be selected by the user.

The approved data set DS2 includes the radiation image P and the imaging failure determination result RS. The imaging failure determination result RS is used as correct answer data to be collated with the imaging failure determination result RS output by the second imaging failure determination device LM2. In some cases, the approved data set DS2 also includes the re-captured image PR in addition to the radiation image P of the first imaging. In this case, a pair of the radiation image P of the first imaging and the imaging failure determination result RS and a pair of the re-captured image PR and the imaging failure determination result RS are each treated as one training data.

The training unit 67 can accumulate the training data, which is the selected approved data set DS2, in the imaging failure image DB 12C. The training unit 67 does not execute the training processing while an accumulated number of the training data is less than a set number set in advance, and executes the training processing of the second imaging failure determination device LM2 in a case in which the accumulated number reaches the set number. Setting information, such as the set number is recorded in the storage device 54, and can be changed as appropriate.

The training unit 67 includes a main processing unit 67A, an evaluation unit 67B, and an update unit 67C. The main processing unit 67A reads the second imaging failure determination device LM2, which is the target of the training processing, from the storage device 54, inputs the radiation image P or the re-captured image PR included in the approved data set DS2 selected as the training data, to the read second imaging failure determination device LM2. Then, the second imaging failure determination device LM2 is caused to execute imaging failure determination processing of outputting the imaging failure determination result RS including the presence or absence of the reason for the imaging failure and the content thereof based on the radiation image P or the re-captured image PR. The basic configuration of the second imaging failure determination device LM2 is the same as the basic configuration of the first imaging failure determination device LM1 described with reference to Fig. 4.

In the main processing unit 67A, the second imaging failure determination device LM2 outputs the imaging failure determination result RS as output data OD to the evaluation unit 67B. The evaluation unit 67B compares the imaging failure determination result RS, which is the output data OD, with correct answer data AD included in the approved data set DS2, which is the training data, and evaluates a difference between the imaging failure determination result RS and the correct answer data AD as a loss by using a loss function. Similar to the first imaging failure determination device LM1, in the second imaging failure determination device LM2, each of the plurality of reasons for the imaging failure is output as the probability. Therefore, in both cases of a case of the loss in a case in which the reason for the imaging failure of the output data OD is an incorrect answer and a case in which the reason for the imaging failure of the output data OD is a correct answer, in a case in which the output value output as the probability is lower than a target value, a difference between the target value and the output value is evaluated as the loss. The evaluation unit 67B outputs the evaluated loss as an evaluation result to the update unit 67C.

The update unit 67C updates values of the filter coefficient in the encoder 46 of the second imaging failure determination device LM2, the weight of the perceptron in the classification unit 47, and the like such that the loss included in the evaluation result is reduced.

The training processing is executed in a case in which the training data are accumulated to be equal to or more than the set number. Therefore, the training processing is performed by batch processing using a plurality of training data. The series of processing from the input to the update of the approved data set DS2, which is the training data, is repeated until an end timing arrives. The end timing is, for example, a case in which all the training of the planned number of the approved data set DS2 ends, a case in which the loss is lower than the target value, and the like.

In a case in which the end timing arrives, the training unit 67 stores a second imaging failure determination device LM2A that is additionally trained in the storage device 54. In this case, the second imaging failure determination device LM2A that is additionally trained is tested by the test processing unit 68.

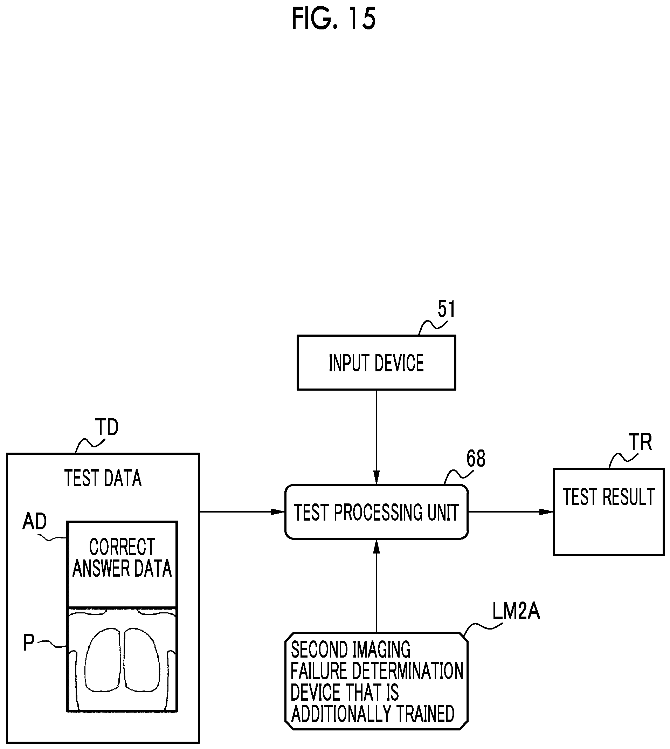

As shown in Fig. 15, the test processing unit 68 executes test processing on the second imaging failure determination device LM2A that is additionally trained. As an example, the test processing is started by an instruction of the user from the input device 51. In the test processing, the test processing unit 68 inputs test data TD to the second imaging failure determination device LM2A, causes the second imaging failure determination device LM2A to execute the imaging failure determination processing, and outputs a test result TR. As described above, the test processing unit 68 executes the test processing using the test data TD for the second imaging failure determination device LM2A for which the training processing is executed, and can perform control of displaying the test result TR on the display 12A.

Similar to the approved data set DS2, the test data TD is composed of a pair of the radiation image P and the imaging failure determination result RS as the correct answer data AD. In the test processing, the second imaging failure determination device LM2A that is additionally trained outputs the imaging failure determination result RS as the test result TR based on the radiation image P of the test data TD. The test data TD is selected from among, for example, data other than the data selected as the training data in the approved data set DS2. The test result TR is determined by, for example, the user. In a case in which the test result TR is not good, the additional training is further executed by the training unit 67. In a case in which the test result TR is good, it is determined that the second imaging failure determination device LM2A that is additionally trained exhibits a target performance, and the additional training ends.

In a case in which the additional training ends, the second imaging failure determination device LM2 that is not additionally trained is substituted with the second imaging failure determination device LM2A that is additionally trained, so that the second imaging failure determination device LM2 is updated.

In a case in which the additional training ends and the second imaging failure determination device LM2 is updated, the update notification unit 69 transmits the update notification to the console 19 of the radiography apparatus 11. That is, in a case in which the training unit 67 executes the training processing for the second imaging failure determination device LM2, the update notification unit 69 transmits the update notification to the console 19. A transmission instruction of the update notification is input through the input device 51 by, for example, the user who confirms the test result TR of the test processing in the imaging failure image management apparatus 12. The update notification is a notification that the first imaging failure determination device LM1 of the console 19 can be updated.

In a case in which the update notification is received, the CPU 32 displays, for example, a message, such as "The first imaging failure determination device LM1 can be updated" on the display 19A. Then, in a case in which the user who views this message inputs an update instruction to the CPU 32 at any timing, the CPU 32 replaces the first imaging failure determination device LM1 with the updated second imaging failure determination device LM2 to execute the update processing of updating the first imaging failure determination device LM1. Of course, in a case in which the CPU 32 receives the update notification, the CPU 32 may execute the update processing of updating the first imaging failure determination device LM1 at an appropriate timing without waiting for the input of the update instruction by the user. As described above, the update notification is a trigger for the radiography apparatus 11 to start the update processing of the first imaging failure determination device LM1 or a notification that can be displayed on the console 19.

The updated second imaging failure determination device LM2 is transmitted from, for example, the imaging failure image management apparatus 12 to the shared folder of the storage device 34 of the console 19. The transmission processing is performed by, for example, the storage processing unit 66 at a timing at which the transmission instruction of the update notification is input from the input device 51. As described above, the storage processing unit 66 stores the second imaging failure determination device LM2 for which the training processing is executed, in the shared folder of the storage device 34 which is the storage unit accessible to the console 19 of the radiography apparatus 11. It should be noted that the console 19 may acquire the second imaging failure determination device LM2 by storing the updated second imaging failure determination device LM2 in the shared folder of the imaging failure image management apparatus 12 and accessing the shared folder from the console 19 side. In addition, the update processing of the first imaging failure determination device LM1 using the second imaging failure determination device LM2 may be performed via a computer, such as a maintenance device of the console 19, other than the imaging failure image management apparatus 12 and the console 19.

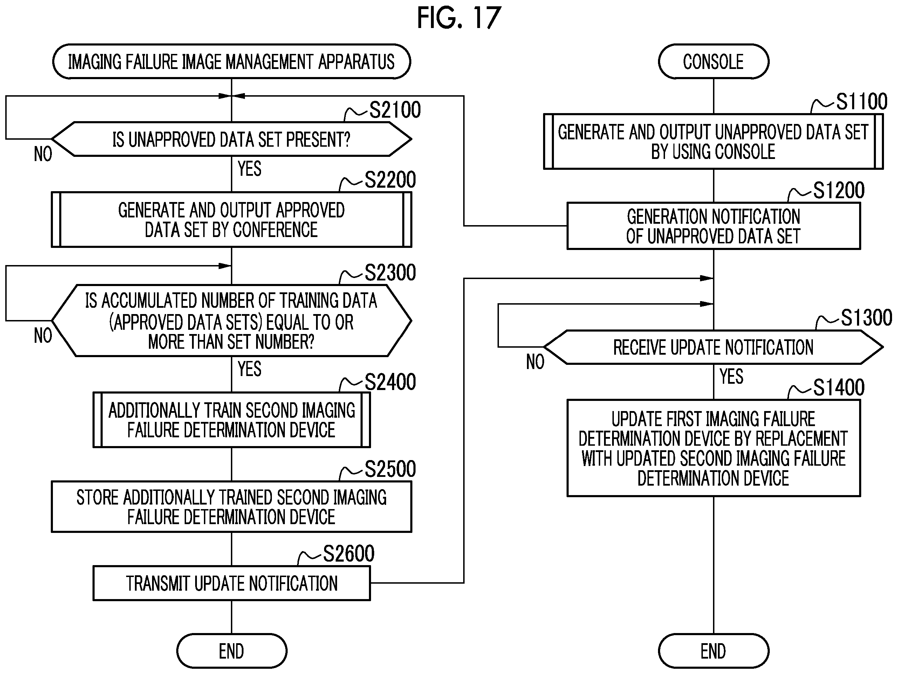

Hereinafter, an action of the configuration described above will be described with reference to the flowcharts shown in Figs. 17 to 20. In a case in which the radiography is performed by using the radiography apparatus 11, in step S1100, the unapproved data set DS1 is generated and output by using the console 19.

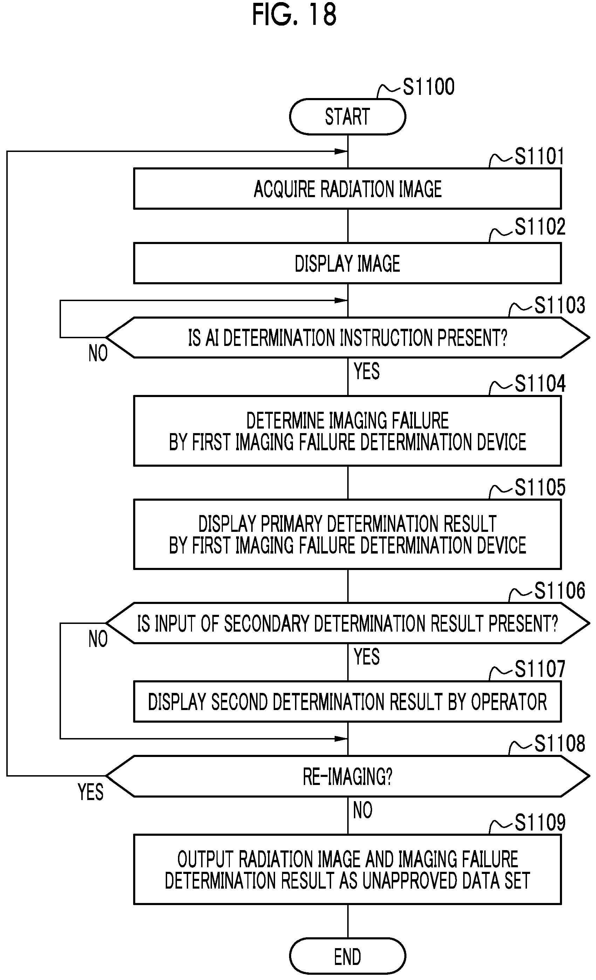

As shown in Fig. 18, in step S1100, the console 19 first acquires the radiation image P captured from the radiation image detection device 18 in step S1101, and displays the acquired radiation image P on the captured image confirmation screen 19B in step S1102 (see Fig. 3). In step S1103, the console 19 waits for the input of an AI determination instruction by the operation of the AI determination button 41 (see Fig. 3). In a case in which the AI determination instruction is input by the operator OP, the console 19 executes the imaging failure determination by the first imaging failure determination device LM1 using the radiation image P as the input data (step S1104). In a case in which the imaging failure determination by the first imaging failure determination device LM1 is executed, the primary determination result RS1, which is the imaging failure determination result RS by the first imaging failure determination device LM1, is output. The output primary determination result RS1 is displayed on the captured image confirmation screen 19B (step S1105). The primary determination result RS1 and the radiation image P are displayed as the unapproved data set DS1.

The operator OP confirms the primary determination result RS1 and the radiation image P, and operates the secondary determination input button 42 (see Fig. 3) as required, such as in a case in which the primary determination result RS1 is determined to be inappropriate, to input the secondary determination result RS2 which is the imaging failure determination result by the operator OP. Since the input of the secondary determination result RS2 is performed optionally by the operator OP, the same reason for the imaging failure may be input in a case in which the determinations of the primary determination result RS1 and the operator OP are the same, or the secondary determination result RS2 may be omitted.

In a case in which the secondary determination result RS2 is input in step S1106, the secondary determination result RS2 by the operator OP is displayed on the captured image confirmation screen 19B in step S1107. In a case in which the secondary determination result RS2 is not input, the processing proceeds to step S1108.

In a case in which the re-imaging is required, the operator OP operates the re-imaging button 43 to perform the re-imaging. In a case in which the re-imaging is performed, the console 19 repeats the processing from step S1101 to step S1107 for the re-captured image PR which is the re-captured radiation image P.

Then, in a case in which the image output button 44 is operated by the operator OP, the unapproved data set DS1 including the radiation image P and the imaging failure determination result RS is output to the image DB 37 and the imaging failure image DB 12C (step S1109).

In Fig. 17, in a case in which the unapproved data set DS1 is generated and output in step S1100, the console 19 transmits a generation notification of the unapproved data set DS1 to the imaging failure image management apparatus 12 in step S1200.

The imaging failure image management apparatus 12 waits for the reception of the generation notification of the unapproved data set DS1 (step S2100). In a case in which the unapproved data set DS1 is present (YES in step S2100), for example, the imaging failure image management apparatus 12 notifies the medical staff ST who is the user by displaying that fact on the display screen. Then, based on the instruction by the medical staff ST, the imaging failure image management apparatus 12 generates and outputs the data set approved by the conference (step S2200).

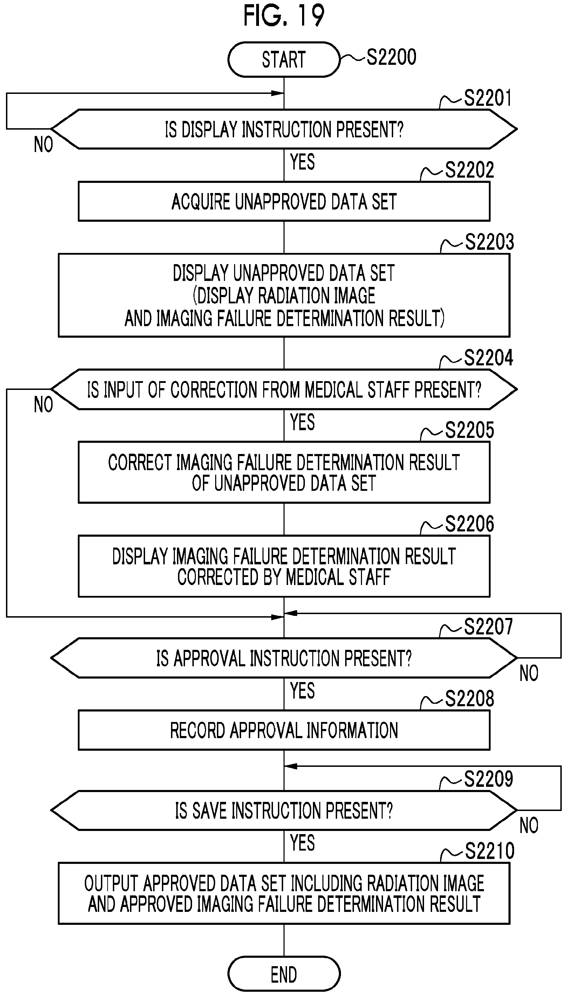

As shown in Fig. 19, in step S2200, the imaging failure image management apparatus 12 waits for the input of the display instruction through the display button 71 (Fig. 9) of the conference screen 12B. In the imaging failure image management apparatus 12, in a case in which the input of the display instruction is received through the display button 71, the data set acquisition unit 61 acquires the unapproved data set DS1 from the imaging failure image DB 12C (step S2202). Then, in step S2203, the display control unit 62 displays the unapproved data set DS1 including the radiation image P and the imaging failure determination result RS on the conference screen 12B (see Fig. 9). In a case in which the unapproved data set DS1 includes the re-captured image PR, the re-captured image PR is also displayed on the conference screen 12B. The plurality of medical staffs ST perform the review on the appropriateness of the imaging failure determination result RS while observing the unapproved data set DS1 displayed on the conference screen 12B of Fig. 9.

In step S2204, the imaging failure image management apparatus 12 waits for the input of the correction instruction by the medical staff ST for the imaging failure determination result RS. Then, in a case in which the correction instruction for the imaging failure determination result RS is input by operating the correction button 73, the correction processing unit 63 corrects the imaging failure determination result RS based on the correction instruction (step S2205). Then, in step S2206, the imaging failure determination result RS corrected by the medical staff ST is displayed. In step S2204, in a case in which the correction instruction is not input, the imaging failure image management apparatus 12 proceeds to step S2207.

In step S2207, the imaging failure image management apparatus 12 waits for the input of the approval instruction by the medical staff ST for the imaging failure determination result RS. Then, in a case in which the approval instruction for the imaging failure determination result RS is input by the operation of the approval button 74, the approval processing unit 64 records the approval information (step S2208). In a case in which the approval information is recorded, the unapproved data set DS1 is changed to the approved data set DS2. The approval information is recorded, as the approval history HSA, in association with the approved data set DS2 including the imaging failure determination result RS. In step 2209, the imaging failure image management apparatus 12 waits for the save instruction by operating the save button 75, and outputs the approved data set DS2 including the radiation image P and the re-captured image PR, and the approved imaging failure determination result RS in a case in which the save instruction is input. The approved data set DS2 is stored in the imaging failure image DB 12C by the storage processing unit 66.

In step S2300 shown in Fig. 17, the imaging failure image management apparatus 12 determines whether or not the accumulated number of the training data selected from the approved data set DS2 is equal to or more than the set number. The training unit 67 does not execute the training processing while the accumulated number of the training data is less than the set number, proceeds to step S2400 in a case in which the accumulated number of the training data is equal to or more than the set number (YES in step S2300), and executes the additional training of the second imaging failure determination device LM2.

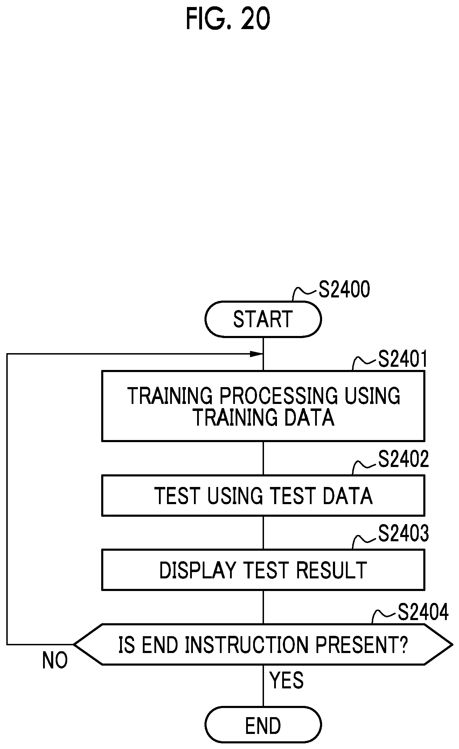

In step S2400 shown in Fig. 20, first, the training unit 67 executes the training processing using the training data selected from the approved data set DS2 (step S2401). In the training processing, as shown in Fig. 14, the additional training of the second imaging failure determination device LM2 is performed. In a case in which the training processing ends, as shown in Fig. 15, the test processing using the test data TD is executed for the second imaging failure determination device LM2 that is additionally trained (step S2402). The test result TR is displayed in a case in which the test processing is executed (step S2403). The test result TR is confirmed by the user. In a case in which the user determines that the test result TR is good, an end instruction of the test processing is input, so that the additional training ends (YES in step S2404). In a case in which the test result TR is not good, the end instruction is not input (step S2404), and the training processing is repeated.

In step S2500 shown in Fig. 17, in a case in which the additional training ends, the second imaging failure determination device LM2A that is additionally trained is stored in the storage device 54. The second imaging failure determination device LM2 in the storage device 54 is updated by the second imaging failure determination device LM2A that is additionally trained. Then, in step S2600, the update notification unit 69 transmits the update notification that the first imaging failure determination device LM1 can be updated by the second imaging failure determination device LM2, to the console 19 of the radiography apparatus 11.

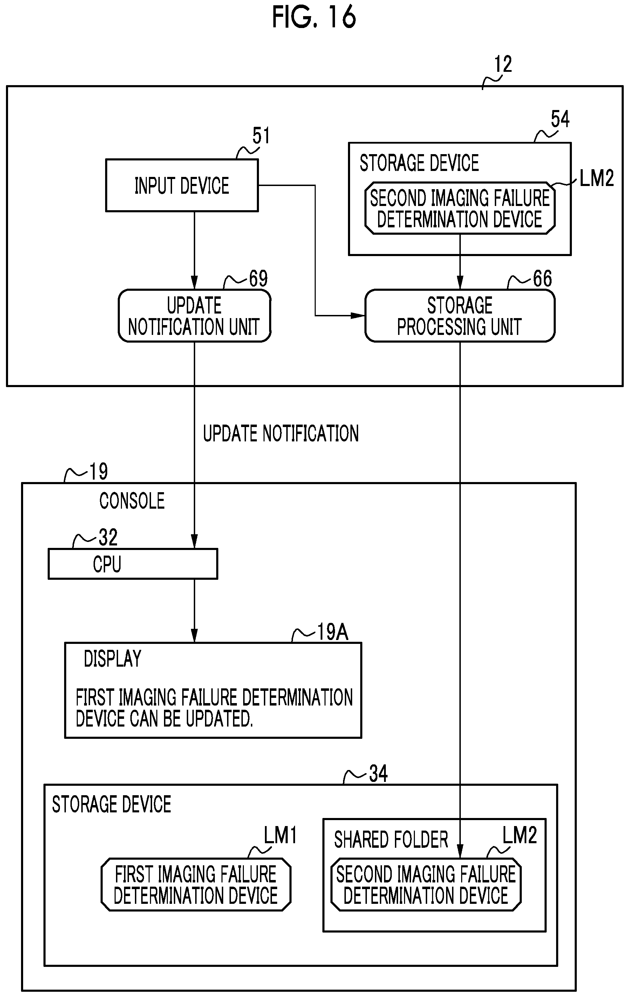

In step S1300, the console 19 waits for the reception of the update notification. Then, in a case in which the update notification is received (YES in step S1300), the console 19 displays the update notification on the display 19A as shown in Fig. 16, and executes the processing of step S1400 based on the instruction of the user. In step S1400, the CPU 32 of the console 19 updates the first imaging failure determination device LM1 by replacing the first imaging failure determination device LM1 with the second imaging failure determination device LM2 updated by the additional training.

As described above, the imaging failure image management apparatus 12 according to the technology of the present disclosure manages at least the imaging failure image among the radiation images (for example, the radiation image P and the re-captured image PR) of the subject captured in the radiography apparatus 11. The processor (for example, a CPU 52) of the imaging failure image management apparatus 12 executes the data set acquisition processing of acquiring the data set (for example, the unapproved data set DS1) including the radiation image input to the first imaging failure determination device (for example, the first imaging failure determination device LM1), which is configured by the machine learning model, performs the imaging failure determination for the radiation image P, and is mounted on the console 19 of the radiography apparatus 11, and the determination result (for example, the imaging failure determination result RS) output from the first imaging failure determination device LM1, and the display control processing of performing control of displaying the data set on the display. Further, the processor executes the correction processing of performing the correction for the imaging failure determination result in a case in which the correction instruction for the determination result of the displayed data set is input, and the training processing of additionally training the second imaging failure determination device (for example, the second imaging failure determination device LM2), which can be substituted with the first imaging failure determination device and is not mounted on the radiography apparatus, by using the data set selected from among the displayed data sets as the training data.

As a result, it is possible to suppress the decrease in the determination accuracy of the first imaging failure determination device mounted on the radiography apparatus. That is, since the imaging failure image management apparatus 12 according to the technology of the present disclosure has the function of displaying the data set, such as the unapproved data set DS1, on the display, for example, it is possible to use the imaging failure image management apparatus 12 in the conference in which the plurality of medical staffs ST, such as the doctor and the technician, review the imaging failure determination result RS. Then, in a case in which such a conference is held, the plurality of medical staffs ST verify the appropriateness of the imaging failure determination result RS included in the displayed data set. In addition, in a case in which the imaging failure determination result RS is incorrect, the imaging failure image management apparatus 12 can correct the imaging failure determination result RS as required. Therefore, the imaging failure determination result RS of the data set displayed in the imaging failure image management apparatus 12 has higher reliability than the independent determination of the operator OP.

Then, the imaging failure image management apparatus 12 executes the training processing of training the second imaging failure determination device, which can be substituted with the first imaging failure determination device, by using the data set selected from among the displayed data sets as the training data. Therefore, the first imaging failure determination device can be updated by additionally training the second imaging failure determination device by using the training data having higher reliability than the related art, and replacing the second imaging failure determination device with the second imaging failure determination device that is additionally trained. Therefore, according to the technology of the present disclosure, it is possible to further suppress the decrease in the determination accuracy of the first imaging failure determination device mounted on the radiography apparatus as compared with the related art in which the first imaging failure determination device is trained by using the training data in which the determination result is corrected by the independent determination of the operator.

In addition, as shown in Fig. 9 and the like, in a case in which the unapproved data set DS1, which is an example of the data set, includes both the primary determination result RS1 output by the first imaging failure determination device and the secondary determination result RS2 by the operator OP, the display control unit 62 of the CPU 52 displays the primary determination result RS1 and the secondary determination result RS2 in a distinguishable manner in the display control processing. As a result, it is possible to verify the appropriateness of each of the primary determination result RS1 and the secondary determination result RS2 in a distinguishable manner.

In the CPU 52, in a case in which the approval instruction by the user is input for the imaging failure determination result RS of the unapproved data set DS1, which is an example of the displayed data set, the approval processing unit 64 records the approval information (for example, the approval history HSA) indicating that the approval is made, in association with the data set which is the approval target. By recording the approval information in association with the data set, it is easy to search for the approved data set. In addition, since the fact that the approval is made in the conference or the like is clarified by recording the approval information, it is easier to search for the data set having high reliability than in a case in which the approval information is not present.

In addition, the approval information means the information indicating that the review by the user is performed for the imaging failure determination result RS which is an example of the determination result. The approved data set is the data set which is the review target, and the approval information is an example of the information indicating that the review is performed. That is, in a case in which the imaging failure determination result RS is reviewed by the user, the approval processing unit 64 records the information (for example, the approval information) indicating the review is performed, in association with the unapproved data set DS1 which is the review target. Since it is considered that the determination result of the data set for which the review is performed has high reliability, it is easy to search for the data set having high reliability in a case in which the information indicating that the review is performed is recorded.

It should be noted that, in the present example, the approval information is shown as an example of the information indicating that the review is performed. However, the approval information and the information indicating that the review is performed may be distinguished from each other. Both the approval information and the information indicating that the review is performed may be recorded, or only one thereof may be recorded. The approval information is preferable as the information indicating the basis for the data set having high reliability, but even only the information indicating that the review is performed is effective as the information indicating the basis for the data set having high reliability. In a case in which the information indicating that the review is performed is recorded, for example, in addition to or instead of the approval button 74, an operation button for inputting a review record is provided on the conference screen 12B of the imaging failure image management apparatus 12. Then, in a case in which the operation button is operated, the CPU 52 records the information indicating that the review is performed, in association with the data set.

In addition, instead of the approval information and the information indicating that the review is performed, a display history indicating that the data set is displayed on the display screen of the imaging failure image management apparatus 12 may be simply recorded. In a case in which the data set is displayed on the imaging failure image management apparatus 12, there is a high possibility that the review is performed on the determination result included in the data set in the conference or the like. In the imaging failure image management apparatus 12, in a case in which the data set, such as the unapproved data set DS1, is displayed on the display screen, such as the conference screen 12B, the CPU 52 records the information indicating that the data set is displayed, in association with the data set. The CPU 52 records the information indicating that the data set is displayed, for example, as the display history. In the display history, for example, the ID information of the data set and the display date and time are recorded, in the same manner as in the approval history HSA.

That is, in the example described above, although the description is made in which the training data is selected from the approved data set DS2, the selection source of the training data does not have to be the approved data set DS2. Specifically, the selection source of the training data may be the data set in which the information indicating that the review is performed is recorded, and further need only be at least the data set having the record being displayed in the imaging failure image management apparatus 12.

In addition, the CPU 52 can accumulate the approved data set DS2, which is an example of the training data, in the imaging failure image DB 12C, which is an example of the storage unit. As shown in the present example, by accumulating the training data, the training processing of the second imaging failure determination device LM2 can be performed by the batch processing using the plurality of training data. Performing the training processing by the batch processing has the following effects.

First, the batch processing can reduce the number of executions of the training processing as compared with the sequential processing in which the training processing is executed each time one training data is generated, so that it is possible to suppress the processing load.

Secondly, it is also possible to reduce the adverse effect on the determination accuracy of the second imaging failure determination device LM2 in the batch processing as compared with the sequential processing. This is because, in a case of the sequential processing, the training data is processed each time one case of the training data is generated. Therefore, for example, in a case in which the training data having the same reason for the imaging failure is continuous, the parameter is optimized for the reason for the imaging failure, and thus the determination accuracy of other reasons for the imaging failure may be decreased. For example, in a case in which the training data having the reason for the imaging failure, such as the internal rotation of the knee joint, is continuous, the determination accuracy of the reason for the imaging failure, such as the insufficient dose, is decreased. As described above, in a case in which the training data is biased, the adverse effect of the determination accuracy is likely to be generated by performing the sequential processing. In the batch processing, since the number of training data is large, there is a high possibility that the bias of the training data is reduced. Therefore, the adverse effect of the determination accuracy can be suppressed as compared with the sequential processing.

In addition, in the sequential processing, for example, in a case in which one case of the training data is specific training data that significantly deviates from the average feature of the training data that is already learned, the training processing is performed by using only the specific training data, and thus there is a concern that the determination accuracy may be adversely affected. In a case of the batch processing, there is a high possibility that the adverse effect of the determination accuracy can be suppressed by averaging the features of the specific training data with the other training data. For these reasons, the batch processing is preferable to the sequential processing. It should be noted that, in the present example, the batch processing is described, but the sequential processing may be performed.

In addition, as shown in Figs. 16 and 17, in a case in which the training processing is executed for the second imaging failure determination device LM2, the CPU 52 transmits the update notification indicating that the first imaging failure determination device LM1 can be updated by the second imaging failure determination device LM2 to the console 19 of the radiography apparatus 11. In the console 19, by receiving the update notification, the update timing of the first imaging failure determination device LM1 can be appropriately determined on the radiography apparatus 11 side.

In addition, the update notification is the trigger for the radiography apparatus 11 to start the update processing of the first imaging failure determination device LM1, or the notification that can be displayed on the console 19 of the radiography apparatus 11. The console 19 of the radiography apparatus 11 can update the first imaging failure determination device LM1 at an appropriate update timing by starting the update processing with the update notification as the trigger. In addition, in a case in which the update notification is the notification that can be displayed on the console 19, the user can update the first imaging failure determination device LM1 at an appropriate timing.

The CPU 52 stores the second imaging failure determination device LM2 for which the training processing of the additional training is executed in the storage unit accessible to the radiography apparatus 11. As shown in Fig. 16, the storage unit is, for example, the shared folder of the storage device 34 of the console 19 shown in Fig. 16. In a case in which the second imaging failure determination device LM2 is stored in the storage unit accessible to the radiography apparatus 11, the update can be performed at an appropriate timing on the radiography apparatus 11 side.

It should be noted that the aspect in which the first imaging failure determination device LM1 is updated by the second imaging failure determination device LM2 is not limited to this, and an aspect may be adopted in which the CPU 52 of the imaging failure image management apparatus 12 accesses the first imaging failure determination device LM1 of the radiography apparatus 11 to forcibly update the first imaging failure determination device LM1. In addition, the first imaging failure determination device LM1 may be updated by using a maintenance device other than the console 19 and the imaging failure image management apparatus 12.

The CPU 52 executes the test processing using the test data for the second imaging failure determination device LM2 for which the training processing of the additional training is executed, and can perform control of displaying the test result TR on the display 12A. Since the imaging failure image management apparatus 12 has the function of testing the second imaging failure determination device LM2 by using the test data, it is possible to provide the second imaging failure determination device having high determination accuracy as the update target as compared with a case in which the test processing is not executed.

The second imaging failure determination device LM2 immediately before the training processing of the additional training is executed is the same as the first imaging failure determination device LM1, and the CPU 52 executes the additional training for the second imaging failure determination device LM2. As a result, on the imaging failure image management apparatus 12 side, it is also easy to understand the performance, such as the state of the first imaging failure determination device LM1 operated in the radiography apparatus 11 and a degree of the determination accuracy improved by the additional training.

It should be noted that the first imaging failure determination device LM1 and the second imaging failure determination device LM2 may be not the same as each other, and the second imaging failure determination device LM2 may be an improved version of the first imaging failure determination device LM1.

It should be noted that, in the example described above, although the description is made in which the test processing is executed after the additional training of the second imaging failure determination device LM2 is performed, the test processing does not have to be executed. Of course, it is preferable to execute the test processing in the meaning that the quality of the second imaging failure determination device LM2 is surely ensured.

Further, in the example described above, an example is described in which the imaging failure image management apparatus 12 acquires all the unapproved data sets DS1 in the imaging failure image DB 12C as the review targets in the conference, but a part of the unapproved data sets DS1 may be acquired, and the acquired part may be used as the review target. For example, the CPU 52 may acquire the data set including the imaging failure determination result that there is the possibility that the radiation image P is the imaging failure image in at least one of the primary determination result RS1 or the secondary determination result RS2 in the data set indicating the unapproved data set DS1 as an example in the data set acquisition processing.

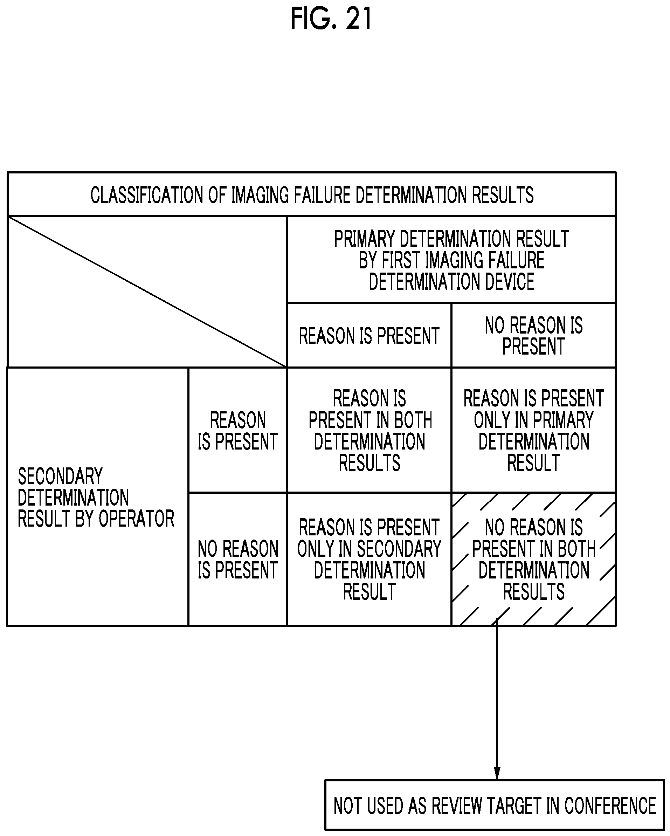

That is, as shown in Fig. 21, the CPU 52 excludes the unapproved data set DS1 in which there is no reason for the imaging failure in both the primary determination result RS1 and the secondary determination result RS2 from the review target at the conference. As a result, in a case in which all the data sets including the radiation image P captured by the radiography apparatus 11 are used as the review targets, the load of the review by the medical staff ST is large. By narrowing down the data set to be acquired, the load of the review can be reduced. In addition, there is a high possibility that the data set in which there is no reason for the imaging failure in both the primary determination result RS1 and the secondary determination result RS2 does not correspond to the imaging failure image. Even in a case in which such a data set is excluded, it is possible to ensure the number of training data having the reason for the imaging failure.

In addition, although the imaging failure determination result RS is indicated by characters in the embodiment described above, the imaging failure determination result RS may be displayed by an image. For example, an image in which a region that is a basis for the reason for the imaging failure in the radiation image P is highlighted may be output. As an example of this image, an image with an annotation indicating the region that is the basis may be used. In addition, as another example, a heat map in which the color or the density is changed depending on a degree of contribution to the determination of the reason for the imaging failure may be used. It should be noted that, in addition to the heat map, the imaging failure determination result RS using characters may be displayed. In addition, the imaging failure determination result RS may include an enlarged image in which the region that is the basis for the reason for the imaging failure in the radiation image P is enlarged. For example, in a case of the internal rotation of the knee joint as the reason for the imaging failure, the enlarged image of the knee joint is included in the imaging failure determination result RS.

In addition, in the embodiment described above, although the description is made in which only the imaging failure image management apparatus 12 has the function of additionally training the second imaging failure determination device LM2 and the radiography apparatus 11 does not have the function of the additional training, the radiography apparatus 11 may have the function of additionally training the first imaging failure determination device LM1. The function of the additional training by the radiography apparatus 11 has a problem as in the related art, but by combining with the imaging failure image management apparatus 12 of the present example, an effect of suppressing the decrease in the determination accuracy can be expected.

In addition, although the radiography apparatus 11 provided in the imaging room is described as an example of the radiography apparatus 11, the radiography apparatus 11 may include a mobile imaging apparatus that can be moved by a carriage.

In addition, the technology of the present disclosure can be applied to a system in which the subject is imaged by using other radiation such as γ-rays, in addition to X-rays.

In each embodiment described above, the hardware structure of the processing unit that executes various processing, such as the data set acquisition unit 61, the display control unit 62, the correction processing unit 63, the approval processing unit 64, the storage processing unit 66, the training unit 67, the test processing unit 68, and the update notification unit 69, is the following various processors.

A CPU, a programmable logic device (PLD), a dedicated electric circuit, and the like are included in various processors. The CPU is a general-purpose processor that executes software (program) and functions as various processing units, as is well known. The PLD is a processor of which a circuit configuration can be changed after manufacturing, such as a field programmable gate array (FPGA). The dedicated electric circuit is a processor having a circuit configuration specially designed for executing specific processing, such as an application specific integrated circuit (ASIC). Similar to the memory connected to the CPU via the data bus, the memory may be connected to the processor or may be built in the processor itself, such as the FPGA.

One processing unit may be configured by using one of these various processors, or may be configured by using a combination of two or more same type or different types of processors (for example, a plurality of FPGAs, or a combination of a CPU and an FPGA). In addition, a plurality of the processing units may be configured by using one processor.

As an example in which a plurality of processing units are configured by using one processor, first, there is a form in which one processor is configured by using a combination of one or more CPUs and the software, and the processor functions as a plurality of processing units. Second, there is a form in which a processor, which realizes the functions of the entire system including the plurality of processing units with one IC chip, is used, as represented by a system on chip (SoC) or the like. As described above, various processing units are configured by using one or more of the various processors described above, as the hardware structure.

Further, the hardware structure of these various processors is, more specifically, an electric circuit (circuitry) in which circuit elements, such as semiconductor elements, are combined.

## Claims

1. An imaging failure image management apparatus that manages at least an imaging failure image among radiation images of a subject captured by a radiography apparatus, the imaging failure image management apparatus comprising:
a processor (52); and
a memory (33) built in or connected to the processor,
wherein the processor executes
data set acquisition processing of acquiring a first, unapproved, data set (DS1) including the radiation image input to a first imaging failure determination device, which is configured by a machine learning model, performs an imaging failure determination for the radiation image, and is mounted on the radiography apparatus, and a determination result output including the result of the imaging failure determination from the first imaging failure determination device and input from an operator (OP) in a case in which the operator determines the result of the imaging failure determination from the first imaging failure determination device to be incorrect,
display control processing of performing control of displaying the data set on a display,
correction processing of performing a correction for the determination result in a case in which a correction instruction for the determination result of the displayed data set is input,
approval processing of changing the first data set (DS1) to a second, approved, data set (DS2) in accordance with an approval instruction that is input in a case in which personnel who have reviewed the image and the determination result verify the appropriateness of the determination result;
storage processing of storing the approved data set (DS2) and approval history (HSA) in an imaging failure image data base (DB) (12C) in a case in which a save instruction is input from the user;
training processing of training a second imaging failure determination device, which is not mounted on the radiography apparatus, by using the approved data set selected by a training unit (67) from the imaging failure image data base (DB) (12C) as the training data based on the approval history (HSA); and
wherein, in a case in which the training processing is executed for the second imaging failure determination device, the processor transmits an update notification indicating that the first imaging failure determination device is updatable by the second imaging failure determination device to the radiography apparatus, the first image failure determination device being updated by replacing it with the trained second image failure determination device.

2. The imaging failure image management apparatus according to claim 1,
wherein the determination result output by the first imaging failure determination device is a primary unapproved data determination result, and the determination result by the operator of the radiography apparatus is a secondary unapproved data determination result, and
the processor displays the primary determination result and the secondary determination result in a distinguishable manner in the display control processing.

3. The imaging failure image management apparatus according to claim 2,
wherein the processor acquires a data set including an imaging failure determination result that there is a possibility that the radiation image corresponds to the imaging failure image in at least one of the primary determination result or the secondary determination result in the data set in the data set acquisition processing.

4. The imaging failure image management apparatus according to any one of claims 1 to 3,
wherein the processor records approval information indicating that approval is made by the personnel.

5. The imaging failure image management apparatus according to any one of claims 1 to 4,
wherein the processor records information indicating that the review by personnel is performed, in association with the data set which is a review target.

6. The imaging failure image management apparatus according to any one of claims 1 to 5,
wherein the processor is able to accumulate the training data in a storage unit (34).

7. The imaging failure image management apparatus according to any one of claims 1 to 6,
wherein the processor does not execute the training processing while an accumulated number of the training data is less than a set number set in advance, and executes the training processing in a case in which the accumulated number reaches the set number.

8. The imaging failure image management apparatus according to any one of claims 1 to 7,
wherein, in a case in which the training processing is executed for the second imaging failure determination device, the processor transmits an update notification indicating that the first imaging failure determination device is updatable by the second imaging failure determination device to the radiography apparatus.

9. The imaging failure image management apparatus according to claim 8,
wherein the update notification is a trigger for the radiography apparatus to start update processing of the first imaging failure determination device, or a notification that is displayable on a console of the radiography apparatus.

10. The imaging failure image management apparatus according to any one of claims 1 to 9,
wherein the processor stores the second imaging failure determination device for which the training processing is executed in a storage unit accessible to the radiography apparatus.

11. The imaging failure image management apparatus according to any one of claims 1 to 10,
wherein the processor
executes test processing using test data for the second imaging failure determination device for which the training processing is executed, and
is able to perform control of displaying a test result on the display.

12. The imaging failure image management apparatus according to any one of claims 1 to 11,
wherein the second imaging failure determination device immediately before the training processing is executed has the same configuration as the first imaging failure determination device, and
the processor executes the training processing for the second imaging failure determination device.

13. An operation method of an imaging failure image management apparatus that manages at least an imaging failure image among radiation images of a subject captured by a radiography apparatus, the operation method comprising:
data set acquisition processing of acquiring a first, unapproved, data set (DS1) including the radiation image input to a first imaging failure determination device, which is configured by a machine learning model, performs an imaging failure determination for the radiation image, and is mounted on the radiography apparatus, and a determination result output including the result of the imaging failure determination from the first imaging failure determination device and input from an operator (OP) in a case in which the operator determines the result of the imaging failure determination from the first imaging failure determination device to be incorrect;
display control processing of performing control of displaying the data set on a display;
correction processing of performing a correction for the determination result in a case in which a correction instruction for the determination result of the displayed data set is input;
approval processing of changing the first data set (DS1) to a second, approved, data set (DS2) in accordance with an approval instruction that is input in a case in which personnel who have reviewed the image and the determination result verify the appropriateness of the determination result;
storage processing of storing the approved data set (DS2) and approval history (HSA) in an imaging failure image data base (DB) (12C) in a case in which a save instruction is input from the user;
training processing of training a second imaging failure determination device, which is not mounted on the radiography apparatus, by using the approved data set selected by a training unit (67) from the imaging failure image data base (DB) (12C) as the training data based on the approval history (HSA); and
wherein, in a case in which the training processing is executed for the second imaging failure determination device, the processor transmits an update notification indicating that the first imaging failure determination device is updatable by the second imaging failure determination device to the radiography apparatus, the first image failure determination device being updated by replacing it with the trained second image failure determination device.

14. A computer-readable storage medium storing an operation program causing a computer to execute:
data set acquisition processing of acquiring a first, unapproved, data set (DS1) including the radiation image input to a first imaging failure determination device, which is configured by a machine learning model, performs an imaging failure determination for the radiation image, and is mounted on the radiography apparatus, and a determination result output including the result of the imaging failure determination from the first imaging failure determination device and input from an operator (OP) in a case in which the operator determines the result of the imaging failure determination from the first imaging failure determination device to be incorrect;
display control processing of performing control of displaying the data set on a display;
correction processing of performing a correction for the determination result in a case in which a correction instruction for the determination result of the displayed data set is input;
approval processing of changing the first data set (DS1) to a second, approved, data set (DS2) in accordance with an approval instruction that is input in a case in which personnel who have reviewed the image and the determination result verify the appropriateness of the determination result;
storage processing of storing the approved data set (DS2) and approval history (HSA) in an imaging failure image data base (DB) (12C) in a case in which a save instruction is input from the user; and
training processing of training a second imaging failure determination device, which is not mounted on the radiography apparatus, by using the approved data set selected by a training unit (67) from the imaging failure image data base (DB) (12C) as the training data based on the approval history (HSA); and
wherein, in a case in which the training processing is executed for the second imaging failure determination device, the processor transmits an update notification indicating that the first imaging failure determination device is updatable by the second imaging failure determination device to the radiography apparatus, the first image failure determination device being updated by replacing it with the trained second image failure determination device.

15. The computer-readable storage medium according to claim 14, wherein the operation program causes the computer to function as the imaging failure image management apparatus that manages at least an imaging failure image among radiation images of a subject captured by the radiography apparatus.

16. A radiography system comprising:
the imaging failure image management apparatus according to any one of claims 1 to 12; and
the radiography apparatus.

## Patentansprüche

1. Bildgebungsfehlerbild-Verwaltungsvorrichtung, die mindestens ein Bildgebungsfehlerbild unter Strahlungsbildern eines Untersuchungsobjekts, die von einer Röntgenvorrichtung aufgenommen worden sind, verwaltet, wobei die Bildgebungsfehlerbild-Verwaltungsvorrichtung umfasst:
einen Prozessor (52); und
einen Speicher (33), der in den Prozessor eingebaut oder mit diesem verbunden ist,
wobei der Prozessor ausführt:
Datensatz-Erfassungsverarbeitung des Erfassens eines ersten, nicht genehmigten,
Datensatzes (DS1), der das Strahlungsbild, das in eine erste Bildgebungsfehler-Bestimmungsvorrichtung eingegeben wird, die durch ein Modell für maschinelles Lernen konfiguriert ist, eine Bildgebungsfehlerbestimmung für das Strahlungsbild durchführt und an der Röntgenvorrichtung montiert ist, und eine Bestimmungsergebnisausgabe, die das Ergebnis der Bildgebungsfehlerbestimmung von der ersten Bildgebungsfehler-Bestimmungsvorrichtung und Eingabe von einem Bediener (OP) in einem Fall enthält, in dem der Bediener bestimmt, dass das Ergebnis der Bildgebungsfehlerbestimmung von der ersten Bildgebungsfehler-Bestimmungsvorrichtung falsch ist, enthält;
Anzeigesteuerverarbeitung des Durchführens von Steuerung des Anzeigens des Datensatzes auf einer Anzeige;
Korrekturverarbeitung des Durchführens einer Korrektur für das Bestimmungsergebnis in einem Fall, in dem eine Korrekturanweisung für das Bestimmungsergebnis des angezeigten Datensatzes eingegeben wird;
Genehmigungsverarbeitung des Änderns des ersten Datensatzes (DS1) in einen zweiten, genehmigten, Datensatz (DS2) in Übereinstimmung mit einer Genehmigungsanweisung, die in einem Fall eingegeben wird, in dem Personal, das das Bild und das Bestimmungsergebnis überprüft hat, die Angemessenheit des Bestimmungsergebnisses verifiziert;
Speicherverarbeitung des Speicherns des genehmigten Datensatzes (DS2) und der Genehmigungshistorie (HSA) in einer Bildgebungsfehlerbild-Datenbank (DB) (12C) in einem Fall, in dem eine Speicheranweisung von dem Benutzer eingegeben wird;
Trainingsverarbeitung des Trainierens einer zweiten Bildgebungsfehler-Bestimmungsvorrichtung, die nicht an der Röntgenvorrichtung montiert ist, durch Verwenden des genehmigten Datensatzes, der von einer Trainingseinheit (67) aus der Bildgebungsfehlerbild-Datenbank (DB) (12C) ausgewählt worden ist, als die Trainingsdaten auf der Grundlage der Genehmigungshistorie (HSA); und
wobei in einem Fall, in dem die Trainingsverarbeitung für die zweite Bildgebungsfehler-Bestimmungsvorrichtung ausgeführt wird, der Prozessor eine Aktualisierungsbenachrichtigung, die angibt, dass die erste Bildgebungsfehler-Bestimmungsvorrichtung durch die zweite Bildgebungsfehler-Bestimmungsvorrichtung aktualisierbar ist, an die Röntgenvorrichtung überträgt, wobei die erste Bildgebungsfehler-Bestimmungsvorrichtung aktualisiert wird, indem sie durch die trainierte zweite Bildgebungsfehler-Bestimmungsvorrichtung ersetzt wird.

2. Bildgebungsfehlerbild-Verwaltungsvorrichtung nach Anspruch 1,
wobei das von der ersten Bildgebungsfehler-Bestimmungsvorrichtung ausgegebene Bestimmungsergebnis ein primäres nicht genehmigtes Datenbestimmungsergebnis ist und das Bestimmungsergebnis von dem Bediener der Röntgenvorrichtung ein sekundäres nicht genehmigtes Datenbestimmungsergebnis ist, und
der Prozessor das primäre Bestimmungsergebnis und das sekundäre Bestimmungsergebnis in der Anzeigesteuerverarbeitung auf unterscheidbare Weise anzeigt.

3. Bildgebungsfehlerbild-Verwaltungsvorrichtung nach Anspruch 2,
wobei der Prozessor einen Datensatz, der ein Bildgebungsfehler-Bestimmungsergebnis dahingehend enthält, dass es eine Möglichkeit gibt, dass das Strahlungsbild dem Bildgebungsfehlerbild entspricht, in mindestens einem von dem primären Bestimmungsergebnis und dem sekundären Bestimmungsergebnis in dem Datensatz bei der Datensatz-Erfassungsverarbeitung erfasst.

4. Bildgebungsfehlerbild-Verwaltungsvorrichtung nach einem der Ansprüche 1 bis 3, wobei der Prozessor Genehmigungsinformationen aufzeichnet, die angeben, dass Genehmigung durch das Personal erfolgt.

5. Bildgebungsfehlerbild-Verwaltungsvorrichtung nach einem der Ansprüche 1 bis 4, wobei der Prozessor Informationen aufzeichnet, die angeben, dass die Überprüfung durch Personal durchgeführt wird, in Verknüpfung mit dem Datensatz, der ein Überprüfungsziel ist.

6. Bildgebungsfehlerbild-Verwaltungsvorrichtung nach einem der Ansprüche 1 bis 5,
wobei der Prozessor in der Lage ist, die Trainingsdaten in einer Speichereinheit (34) zu akkumulieren.

7. Bildgebungsfehlerbild-Verwaltungsvorrichtung nach einem der Ansprüche 1 bis 6,
wobei der Prozessor die Trainingsverarbeitung nicht ausführt, während eine akkumulierte Anzahl der Trainingsdaten kleiner als eine eingestellte Anzahl ist, die im Voraus eingestellt ist, und die Trainingsverarbeitung in einem Fall ausführt, in dem die akkumulierte Anzahl die eingestellte Anzahl erreicht.

8. Bildgebungsfehlerbild-Verwaltungsvorrichtung nach einem der Ansprüche 1 bis 7,
wobei in einem Fall, in dem die Trainingsverarbeitung für die zweite Bildgebungsfehler-Bestimmungsvorrichtung ausgeführt wird, der Prozessor eine Aktualisierungsbenachrichtigung, die angibt, dass die erste Bildgebungsfehler-Bestimmungsvorrichtung durch die zweite Bildgebungsfehler-Bestimmungsvorrichtung aktualisierbar ist, an die Röntgenvorrichtung überträgt.

9. Bildgebungsfehlerbild-Verwaltungsvorrichtung nach Anspruch 8,
wobei die Aktualisierungsbenachrichtigung ein Auslöser für die Röntgenvorrichtung, um Aktualisierungsverarbeitung der ersten Bildgebungsfehler-Bestimmungsvorrichtung zu starten, oder eine Benachrichtigung ist, die auf einer Konsole der Röntgenvorrichtung anzeigbar ist.

10. Bildgebungsfehlerbild-Verwaltungsvorrichtung nach einem der Ansprüche 1 bis 9,
wobei der Prozessor die zweite Bildgebungsfehler-Bestimmungsvorrichtung, für die die Trainingsverarbeitung ausgeführt wird, in einer Speichereinheit speichert, die für die Röntgenvorrichtung zugänglich ist.

11. Bildgebungsfehlerbild-Verwaltungsvorrichtung nach einem der Ansprüche 1 bis 10,
wobei der Prozessor
Testverarbeitung unter Verwendung von Testdaten für die zweite Bildgebungsfehler-Bestimmungsvorrichtung, für die die Trainingsverarbeitung ausgeführt wird, ausführt, und
in der Lage ist, Steuerung des Anzeigens eines Testergebnisses auf der Anzeige durchzuführen.

12. Bildgebungsfehlerbild-Verwaltungsvorrichtung nach einem der Ansprüche 1 bis 11,
wobei die zweite Bildgebungsfehler-Bestimmungsvorrichtung, unmittelbar bevor die Trainingsverarbeitung ausgeführt wird, die gleiche Konfiguration wie die erste Bildgebungsfehler-Bestimmungsvorrichtung aufweist, und
der Prozessor die Trainingsverarbeitung für die zweite Bildgebungsfehler-Bestimmungsvorrichtung ausführt.

13. Betriebsverfahren einer Bildgebungsfehlerbild-Verwaltungsvorrichtung, die mindestens ein Bildgebungsfehlerbild unter Strahlungsbildern eines Untersuchungsobjekts verwaltet, die von einer Röntgenvorrichtung aufgenommen worden sind, wobei das Betriebsverfahren umfasst:
Datensatz-Erfassungsverarbeitung des Erfassens eines ersten, nicht genehmigten, Datensatzes (DS1), der das Strahlungsbild, das in eine erste Bildgebungsfehler-Bestimmungsvorrichtung eingegeben wird, die durch ein Modell für maschinelles Lernen konfiguriert ist, eine Bildgebungsfehlerbestimmung für das Strahlungsbild durchführt und an der Röntgenvorrichtung montiert ist, und eine Bestimmungsergebnisausgabe, die das Ergebnis der Bildgebungsfehlerbestimmung von der ersten Bildgebungsfehler-Bestimmungsvorrichtung und Eingabe von einem Bediener (OP) in einem Fall enthält, in dem der Bediener bestimmt, dass das Ergebnis der Bildgebungsfehlerbestimmung von der ersten Bildgebungsfehler-Bestimmungsvorrichtung falsch ist, enthält;
Anzeigesteuerverarbeitung des Durchführens von Steuerung des Anzeigens des Datensatzes auf einer Anzeige;
Korrekturverarbeitung des Durchführens einer Korrektur für das Bestimmungsergebnis in einem Fall, in dem eine Korrekturanweisung für das Bestimmungsergebnis des angezeigten Datensatzes eingegeben wird;
Genehmigungsverarbeitung des Änderns des ersten Datensatzes (DS1) in einen zweiten, genehmigten, Datensatz (DS2) in Übereinstimmung mit einer Genehmigungsanweisung, die in einem Fall eingegeben wird, in dem Personal, das das Bild und das Bestimmungsergebnis überprüft hat, die Angemessenheit des Bestimmungsergebnisses verifiziert;
Speicherverarbeitung des Speicherns des genehmigten Datensatzes (DS2) und der Genehmigungshistorie (HSA) in einer Bildgebungsfehlerbild-Datenbank (DB) (12C) in einem Fall, in dem eine Speicheranweisung von dem Benutzer eingegeben wird;
Trainingsverarbeitung des Trainierens einer zweiten Bildgebungsfehler-Bestimmungsvorrichtung, die nicht an der Röntgenvorrichtung montiert ist, durch Verwenden des genehmigten Datensatzes, der von einer Trainingseinheit (67) aus der Bildgebungsfehlerbild-Datenbank (DB) (12C) ausgewählt worden ist, als die Trainingsdaten auf der Grundlage der Genehmigungshistorie (HSA); und
wobei in einem Fall, in dem die Trainingsverarbeitung für die zweite Bildgebungsfehler-Bestimmungsvorrichtung ausgeführt wird, der Prozessor eine Aktualisierungsbenachrichtigung, die angibt, dass die erste Bildgebungsfehler-Bestimmungsvorrichtung durch die zweite Bildgebungsfehler-Bestimmungsvorrichtung aktualisierbar ist, an die Röntgenvorrichtung überträgt, wobei die erste Bildgebungsfehler-Bestimmungsvorrichtung aktualisiert wird, indem sie durch die trainierte zweite Bildgebungsfehler-Bestimmungsvorrichtung ersetzt wird.

14. Computerlesbares Speichermedium, das ein Betriebsprogramm speichert, das einen Computer veranlasst, auszuführen:
Datensatz-Erfassungsverarbeitung des Erfassens eines ersten, nicht genehmigten,
Datensatzes (DS1), der das Strahlungsbild, das in eine erste Bildgebungsfehler-Bestimmungsvorrichtung eingegeben wird, die durch ein Modell für maschinelles Lernen konfiguriert ist, eine Bildgebungsfehlerbestimmung für das Strahlungsbild durchführt und an der Röntgenvorrichtung montiert ist, und eine Bestimmungsergebnisausgabe, die das Ergebnis der Bildgebungsfehlerbestimmung von der ersten Bildgebungsfehler-Bestimmungsvorrichtung und Eingabe von einem Bediener (OP) in einem Fall enthält, in dem der Bediener bestimmt, dass das Ergebnis der Bildgebungsfehlerbestimmung von der ersten Bildgebungsfehler-Bestimmungsvorrichtung falsch ist, enthält;
Anzeigesteuerverarbeitung des Durchführens von Steuerung des Anzeigens des Datensatzes auf einer Anzeige;
Korrekturverarbeitung des Durchführens einer Korrektur für das Bestimmungsergebnis in einem Fall, in dem eine Korrekturanweisung für das Bestimmungsergebnis des angezeigten Datensatzes eingegeben wird;
Genehmigungsverarbeitung des Änderns des ersten Datensatzes (DS1) in einen zweiten, genehmigten, Datensatz (DS2) in Übereinstimmung mit einer Genehmigungsanweisung, die in einem Fall eingegeben wird, in dem Personal, das das Bild und das Bestimmungsergebnis überprüft hat, die Angemessenheit des Bestimmungsergebnisses verifiziert;
Speicherverarbeitung des Speicherns des genehmigten Datensatzes (DS2) und der Genehmigungshistorie (HSA) in einer Bildgebungsfehlerbild-Datenbank (DB) (12C) in einem Fall, in dem eine Speicheranweisung von dem Benutzer eingegeben wird; und
Trainingsverarbeitung des Trainierens einer zweiten Bildgebungsfehler-Bestimmungsvorrichtung, die nicht an der Röntgenvorrichtung montiert ist, durch Verwenden des genehmigten Datensatzes, der von einer Trainingseinheit (67) aus der Bildgebungsfehlerbild-Datenbank (DB) (12C) ausgewählt worden ist, als die Trainingsdaten auf der Grundlage der Genehmigungshistorie (HSA); und
wobei in einem Fall, in dem die Trainingsverarbeitung für die zweite Bildgebungsfehler-Bestimmungsvorrichtung ausgeführt wird, der Prozessor eine Aktualisierungsbenachrichtigung, die angibt, dass die erste Bildgebungsfehler-Bestimmungsvorrichtung durch die zweite Bildgebungsfehler-Bestimmungsvorrichtung aktualisierbar ist, an die Röntgenvorrichtung überträgt, wobei die erste Bildgebungsfehler-Bestimmungsvorrichtung aktualisiert wird, indem sie durch die trainierte zweite Bildgebungsfehler-Bestimmungsvorrichtung ersetzt wird.

15. Computerlesbares Speichermedium nach Anspruch 14, wobei das Betriebsprogramm den Computer veranlasst, als die Bildgebungsfehlerbild-Verwaltungsvorrichtung zu fungieren, die mindestens ein Bildgebungsfehlerbild unter Strahlungsbildern eines Untersuchungsobjekts verwaltet, die von der Röntgenvorrichtung aufgenommen worden sind.

16. Röntgensystem, umfassend:
die Bildgebungsfehlerbild-Verwaltungsvorrichtung nach einem der Ansprüche 1 bis 12; und
die Röntgenvorrichtung.

## Revendications

1. Appareil de gestion d'images d'échec d'imagerie qui gère au moins une image d'échec d'imagerie parmi des images de rayonnement d'un sujet capturées par un appareil de radiographie, l'appareil de gestion d'images d'échec d'imagerie comprenant :
un processeur (52) ; et
une mémoire (33) intégrée ou connectée au processeur,
dans lequel le processeur exécute
un traitement d'acquisition d'ensemble de données consistant à acquérir un premier ensemble de données non approuvé (DS1) incluant l'image de rayonnement entrée dans un premier dispositif de détermination d'échec d'imagerie, qui est configuré par un modèle d'apprentissage automatique, effectue une détermination d'échec d'imagerie pour l'image de rayonnement, et est monté sur l'appareil de radiographie, et une sortie de résultat de détermination incluant le résultat de la détermination d'échec d'imagerie provenant du premier dispositif de détermination d'échec d'imagerie et une entrée provenant d'un opérateur (OP) dans un cas où l'opérateur détermine que le résultat de la détermination d'échec d'imagerie provenant du premier dispositif de détermination d'échec d'imagerie est incorrect ;
un traitement de contrôle d'affichage consistant à effectuer un contrôle d'affichage de l'ensemble de données sur un affichage ;
un traitement de correction consistant à effectuer une correction du résultat de détermination dans un cas où une instruction de correction du résultat de détermination de l'ensemble de données affiché est entrée ;
un traitement d'approbation consistant à changer le premier ensemble de données (DS1) en un deuxième ensemble de données approuvé (DS2) conformément à une instruction d'approbation qui est entrée dans un cas où le personnel qui a examiné l'image et le résultat de détermination vérifie la pertinence du résultat de détermination ;
un traitement de stockage consistant à stocker l'ensemble de données approuvé (DS2) et un historique d'approbation (HSA) dans une base de données d'images d'échec d'imagerie (DB) (12C) dans un cas où une instruction d'enregistrement est entrée par l'utilisateur ;
un traitement d'apprentissage consistant à entraîner un deuxième dispositif de détermination d'échec d'imagerie, qui n'est pas monté sur l'appareil de radiographie, en utilisant l'ensemble de données approuvé sélectionné par une unité d'apprentissage (67) dans la base de données d'images d'échec d'imagerie (DB) (12C) en tant que données d'apprentissage sur la base de l'historique d'approbation (HSA) ; et
dans lequel, dans un cas où le traitement d'apprentissage est exécuté pour le deuxième dispositif de détermination d'échec d'imagerie, le processeur transmet, à l'appareil de radiographie, une notification de mise à jour indiquant que le premier dispositif de détermination d'échec d'imagerie peut être mis à jour par le deuxième dispositif de détermination d'échec d'imagerie, le premier dispositif de détermination d'échec d'imagerie étant mis à jour en le remplaçant par le deuxième dispositif de détermination d'échec d'imagerie entraîné.

2. Appareil de gestion d'images d'échec d'imagerie selon la revendication 1,
dans lequel le résultat de détermination sorti par le premier dispositif de détermination d'échec d'imagerie est un résultat de détermination primaire de données non approuvées, et le résultat de détermination par l'opérateur de l'appareil de radiographie est un résultat de détermination secondaire de données non approuvées, et
le processeur affiche le résultat de détermination primaire et le résultat de détermination secondaire d'une manière distinguable dans le traitement de contrôle d'affichage.

3. Appareil de gestion d'images d'échec d'imagerie selon la revendication 2,
dans lequel, dans le traitement d'acquisition d'ensemble de données, le processeur acquiert un ensemble de données incluant un résultat de détermination d'échec d'imagerie indiquant qu'il existe une possibilité que l'image de rayonnement corresponde à l'image d'échec d'imagerie dans au moins l'un du résultat de détermination primaire ou du résultat de détermination secondaire dans l'ensemble de données.

4. Appareil de gestion d'images d'échec d'imagerie selon l'une quelconque des revendications 1 à 3,
dans lequel le processeur enregistre des informations d'approbation indiquant qu'une approbation est effectuée par le personnel.

5. Appareil de gestion d'images d'échec d'imagerie selon l'une quelconque des revendications 1 à 4,
dans lequel le processeur enregistre des informations indiquant que l'examen par le personnel est effectué, en association avec l'ensemble de données qui est une cible d'examen.

6. Appareil de gestion d'images d'échec d'imagerie selon l'une quelconque des revendications 1 à 5,
dans lequel le processeur est capable d'accumuler les données d'apprentissage dans une unité de stockage (34).

7. Appareil de gestion d'images d'échec d'imagerie selon l'une quelconque des revendications 1 à 6,
dans lequel le processeur n'exécute pas le traitement d'apprentissage tant qu'un nombre accumulé des données d'apprentissage est inférieur à un nombre prédéfini, et exécute le traitement d'apprentissage dans un cas où le nombre accumulé atteint le nombre prédéfini.

8. Appareil de gestion d'images d'échec d'imagerie selon l'une quelconque des revendications 1 à 7,
dans lequel, dans un cas où le traitement d'apprentissage est exécuté pour le deuxième dispositif de détermination d'échec d'imagerie, le processeur transmet, à l'appareil de radiographie, une notification de mise à jour indiquant que le premier dispositif de détermination d'échec d'imagerie peut être mis à jour par le deuxième dispositif de détermination d'échec d'imagerie.

9. Appareil de gestion d'images d'échec d'imagerie selon la revendication 8, dans lequel la notification de mise à jour est un déclencheur pour l'appareil de radiographie afin de démarrer un traitement de mise à jour du premier dispositif de détermination d'échec d'imagerie, ou une notification qui est affichable sur une console de l'appareil de radiographie.

10. Appareil de gestion d'images d'échec d'imagerie selon l'une quelconque des revendications 1 à 9,
dans lequel le processeur stocke le deuxième dispositif de détermination d'échec d'imagerie pour lequel le traitement d'apprentissage est exécuté dans une unité de stockage accessible à l'appareil de radiographie.

11. Appareil de gestion d'images d'échec d'imagerie selon l'une quelconque des revendications 1 à 10,
dans lequel le processeur
exécute un traitement de test en utilisant des données de test pour le deuxième dispositif de détermination d'échec d'imagerie pour lequel le traitement d'apprentissage est exécuté, et
est capable d'effectuer un contrôle d'affichage d'un résultat de test sur l'affichage.

12. Appareil de gestion d'images d'échec d'imagerie selon l'une quelconque des revendications 1 à 11,
dans lequel le deuxième dispositif de détermination d'échec d'imagerie, immédiatement avant que le traitement d'apprentissage soit exécuté, présente la même configuration que le premier dispositif de détermination d'échec d'imagerie, et
le processeur exécute le traitement d'apprentissage pour le deuxième dispositif de détermination d'échec d'imagerie.

13. Procédé de fonctionnement d'un appareil de gestion d'images d'échec d'imagerie qui gère au moins une image d'échec d'imagerie parmi des images de rayonnement d'un sujet capturées par un appareil de radiographie, le procédé de fonctionnement comprenant :
un traitement d'acquisition d'ensemble de données consistant à acquérir un premier ensemble de données non approuvé (DS1) incluant l'image de rayonnement entrée dans un premier dispositif de détermination d'échec d'imagerie, qui est configuré par un modèle d'apprentissage automatique, effectue une détermination d'échec d'imagerie pour l'image de rayonnement, et est monté sur l'appareil de radiographie, et une sortie de résultat de détermination incluant le résultat de la détermination d'échec d'imagerie provenant du premier dispositif de détermination d'échec d'imagerie et une entrée provenant d'un opérateur (OP) dans un cas où l'opérateur détermine que le résultat de la détermination d'échec d'imagerie provenant du premier dispositif de détermination d'échec d'imagerie est incorrect ;
un traitement de contrôle d'affichage consistant à effectuer un contrôle d'affichage de l'ensemble de données sur un affichage ;
un traitement de correction consistant à effectuer une correction du résultat de détermination dans un cas où une instruction de correction du résultat de détermination de l'ensemble de données affiché est entrée ;
un traitement d'approbation consistant à changer le premier ensemble de données (DS1) en un deuxième ensemble de données approuvé (DS2) conformément à une instruction d'approbation qui est entrée dans un cas où le personnel qui a examiné l'image et le résultat de détermination vérifie la pertinence du résultat de détermination ;
un traitement de stockage consistant à stocker l'ensemble de données approuvé (DS2) et un historique d'approbation (HSA) dans une base de données d'images d'échec d'imagerie (DB) (12C) dans un cas où une instruction d'enregistrement est entrée par l'utilisateur ;
un traitement d'apprentissage consistant à entraîner un deuxième dispositif de détermination d'échec d'imagerie, qui n'est pas monté sur l'appareil de radiographie, en utilisant l'ensemble de données approuvé sélectionné par une unité d'apprentissage (67) dans la base de données d'images d'échec d'imagerie (DB) (12C) en tant que données d'apprentissage sur la base de l'historique d'approbation (HSA) ; et
dans lequel, dans un cas où le traitement d'apprentissage est exécuté pour le deuxième dispositif de détermination d'échec d'imagerie, le processeur transmet, à l'appareil de radiographie, une notification de mise à jour indiquant que le premier dispositif de détermination d'échec d'imagerie peut être mis à jour par le deuxième dispositif de détermination d'échec d'imagerie, le premier dispositif de détermination d'échec d'imagerie étant mis à jour en le remplaçant par le deuxième dispositif de détermination d'échec d'imagerie entraîné.

14. Support de stockage lisible par ordinateur stockant un programme de fonctionnement amenant un ordinateur à exécuter :
un traitement d'acquisition d'ensemble de données consistant à acquérir un premier ensemble de données non approuvé (DS1) incluant l'image de rayonnement entrée dans un premier dispositif de détermination d'échec d'imagerie, qui est configuré par un modèle d'apprentissage automatique, effectue une détermination d'échec d'imagerie pour l'image de rayonnement, et est monté sur l'appareil de radiographie, et une sortie de résultat de détermination incluant le résultat de la détermination d'échec d'imagerie provenant du premier dispositif de détermination d'échec d'imagerie et une entrée provenant d'un opérateur (OP) dans un cas où l'opérateur détermine que le résultat de la détermination d'échec d'imagerie provenant du premier dispositif de détermination d'échec d'imagerie est incorrect ;
un traitement de contrôle d'affichage consistant à effectuer un contrôle d'affichage de l'ensemble de données sur un affichage ;
un traitement de correction consistant à effectuer une correction du résultat de détermination dans un cas où une instruction de correction du résultat de détermination de l'ensemble de données affiché est entrée ;
un traitement d'approbation consistant à changer le premier ensemble de données (DS1) en un deuxième ensemble de données approuvé (DS2) conformément à une instruction d'approbation qui est entrée dans un cas où le personnel qui a examiné l'image et le résultat de détermination vérifie la pertinence du résultat de détermination ;
un traitement de stockage consistant à stocker l'ensemble de données approuvé (DS2) et un historique d'approbation (HSA) dans une base de données d'images d'échec d'imagerie (DB) (12C) dans un cas où une instruction d'enregistrement est entrée par l'utilisateur ; et
un traitement d'apprentissage consistant à entraîner un deuxième dispositif de détermination d'échec d'imagerie, qui n'est pas monté sur l'appareil de radiographie, en utilisant l'ensemble de données approuvé sélectionné par une unité d'apprentissage (67) dans la base de données d'images d'échec d'imagerie (DB) (12C) en tant que données d'apprentissage sur la base de l'historique d'approbation (HSA) ; et
dans lequel, dans un cas où le traitement d'apprentissage est exécuté pour le deuxième dispositif de détermination d'échec d'imagerie, le processeur transmet, à l'appareil de radiographie, une notification de mise à jour indiquant que le premier dispositif de détermination d'échec d'imagerie peut être mis à jour par le deuxième dispositif de détermination d'échec d'imagerie, le premier dispositif de détermination d'échec d'imagerie étant mis à jour en le remplaçant par le deuxième dispositif de détermination d'échec d'imagerie entraîné.

15. Support de stockage lisible par ordinateur selon la revendication 14, dans lequel le programme de fonctionnement amène l'ordinateur à fonctionner en tant qu'appareil de gestion d'images d'échec d'imagerie qui gère au moins une image d'échec d'imagerie parmi des images de rayonnement d'un sujet capturées par l'appareil de radiographie.

16. Système de radiographie comprenant :
l'appareil de gestion d'images d'échec d'imagerie selon l'une quelconque des revendications 1 à 12 ; et
l'appareil de radiographie.
